# EUROPEAN PATENT SPECIFICATION

(11) **EP 0 478 545 B1**
(45) Date of publication and mention of the grant of the patent: **11.08.1999**
(21) Application number: 89911346.8
(22) Date of filing: 18.09.1989
(51) Int. Cl.: A61K 31/53

(54) **1,2,4-BENZOTRIAZINE OXIDES AS RADIOSENSITIZERS AND SELECTIVE CYTOTOXIC AGENTS**
1,2,4-BENZOTRIAZINOXIDE ALS FUNKEMPFINDLICHE UND SELEKTIVE ZYTOTOXISCHE MITTEL
OXYDES DE 1,2,4-BENZOTRIAZINE A TITRE DE RADIOSENSIBILISATEURS ET D'AGENTS CYTOTOXIQUES SELECTIFS

(43) Date of publication of application: 08.04.1992
(73) Proprietor: SRI INTERNATIONAL, Menlo Park, California 94025-3493 (US)
(72) Inventor: LEE, William, W., Palo Alto, CA 94303 (US); BROWN, J., Martin, Redwood City California 94061 (US); GRANGE, Edward, W., Palo Alto, CA 94306 (US); MARTINEZ, Abelardo, P., San Jose, CA 95129 (US); TRACY, Michael, Menlo Park, CA 94025 (US); POLLART, Daniel, J., Menlo Park, CA 94025 (US)
(74) Representative: Goldin, Douglas Michael
(86) International application number: US8904112
(87) International publication number: WO9104028

(56) References cited:
- WO-A-88/02366
- WO-A-89/08647
- DE-A- 2 404 375
- US-A- 3 079 390
- US-A- 3 980 779
- US-A- 3 991 189
- US-A- 4 001 410
- US-A- 4 022 022
- US-A- 4 027 022
- US-A- 4 091 098
- INT. J. RADIATION ONCOLOGY, BIOL. PHYS., vol. 12, no. 7, 1986, pages 1239-1242, Pergamon Journals Ltd., US; E.M. ZEMAN et al.: "SR-4233: A new bioreductive agent with high selective toxicity for hypoxic mammalian cells"
- TETRAHEDRON, vol. 38, no. 12, 1982, pages 1793-1796, Pergamon Press Ltd., GB; R.H. ATALLAH et al.: "Oxides of 3-methyl-1,2,4-benzotriazine"
- RADIOTHERAPY AND ONCOLOGY, vol. 18, no. 3, 1988, pages 209-218, Elsevier Science Publishers B.V.; E.M. ZEMAN et al.: "Enhancement of radiation-induced tumor cell killing by the hypoxic cell toxin SR 4233"
- BIOCHEMICAL PHARMACOLOGY, vol. 35, no. 19, October 1, 1986, pages 3417-3420; K. R. LADEROUTE et al.: "Identification of two major reduction products of the hypoxic cell toxin 3-amino-1,2,4-benzotriazine-1,4-dioxide"
- PROCEEDINGS OF THE AMERICAN ASSOCIATION FOR CANCER RESEARCH, 18th annual meeting, May 24-27, 1989, San Francisco, Ca. US, vol. 30, March 1989, page 575, no. 2290; M. TRACY et al.: "Structure-activity relationships of 1,2,4- benzotriazine 1,4-di-N-oxides, a new class of bioreductive drugs with tumor specificity"
- INTERNATIONAL JOURNAL OF RADIATION ONCOLOGY BIOLOGY PHYSICS, vol. 16, no. 4, April 1989, pages 977-981; E.M. ZEMAN et al.: "Structure-activity relationships for benzotriazine di-N-oxides"
- 35TH ANN. MEETING OF RADIATION RES. SOC. and 7TH ANN. MEETING OF NORTH AMER. HYPERTHERMIA GROUP, February 21-26, 1987, Atlanta, US, 1986, page 114, no. Fd- 4; J.M. BROWN et al.: "Benzotriazine-N-oxides: A new class of bioreductive cytotoxic agents"

## Description

### Technical Field

The invention relates to cytotoxic agents and radiotherapy effective against hypoxic cells. More specifically, the invention relates to certain novel 1,2,4-benzotriazine oxides, to the use of selected 1,2,4-benzotriazine oxides in killing tumor cells and/or sensitizing tumor cells to radiation, and to novel synthetic methods.

### Background Art

Hypoxic cell radiosensitizers are compounds that selectively increase the sensitivity of hypoxic cells to destructive radiation. Cytotoxins which have enhanced activity under hypoxic conditions also provide a means for selective destruction of cells under low oxygen pressure. This specificity for hypoxic cells is important because it is tumors that are typically characterized by such cells. Virtually all tumors which are present as solid masses contain these cells, while normal cells generally have an adequate supply of oxygen. Accordingly, anti-tumor agents can be made selective for tumors by virtue of high activity under hypoxic conditions, and radiation can be employed more effectively in the presence of these sensitizers.

Of course, the use of radiation treatment to destroy tumor cells is only practical if damage to the surrounding normal tissue can be minimized or avoided. The effects of radiation are enhanced by the presence of oxygen, and it is established that as the dose of radiation is increased, the effectiveness of the radiation in destroying target cells is enhanced most dramatically when oxygen is present. Therefore, selectivity for tumor cells toward radiation is difficult to achieve -- normal cells, in view of their oxygen supply, are generally more susceptible to radiation than the target tumor cells. It is therefore desirable to provide a means of sensitizing tumor cells, but not the surrounding tissue, to radiation treatment. One solution would be to increase the supply of oxygen to these tumor cells. This, however, has proved difficult to do.

Various heterocyclic compounds and in particular those with oxidized nitrogen moieties, have been used to radiosensitize hypoxic tumor cells. Indeed, it has been postulated that the oxidized nitrogen functionality is responsible for this activity. Nitroimidazoles, particularly misonidazole (MIS) and metronidazole have been studied extensively, and MIS is commonly used as a standard in in vitro and in vivo tests for radiosensitizing activity. (See, e.g., Asquith, et al, Radiation Res (1974) 60:108-118; Hall, et al, Brit J Cancer (1978) 37: 567-569; Brown, et al, Radiation Res (1980) 82:171-190; and U.S. patent 4,371,540. The radiosensitizing activities of certain 1-substituted 3(5)-nitro-s-triazoles and of various quinoxaline-1,4-dioxide derivatives have also been disclosed.

In addition, US Serial Nos. 730,761, filed 3 May 1985, and 788,762, filed 18 October 1985 assigned to the same assignee and incorporated by reference disclose a group of radiosensitizers that do not contain oxidized nitrogen -- the substituted benzamides and nicotinamides and their thio analogs. These compounds, nevertheless, are radiosensitizers. It is important to distinguish the ability to sensitize hypoxic cells selectively, for instance, by enhancing their oxygen supply, from another mechanism commonly encountered for "sensitizing" cells: inhibition of the enzyme poly(ADP-ribose)polymerase, which is believed to be essential in the repair of irradiated cells after radiation. This repair mechanism is operative in both hypoxic tumor cells and in normal cells. Hence, administration of "radiosensitizers" which operate according to this latter mechanism does not accomplish the desired purpose of selectively sensitizing the target tumor cells.

A group of compounds which has not previously been suggested for use in either selectively killing hypoxic cells or in radiosensitizing such cells is 3-amino-1,2,4-benzotriazine 1,4-di-N-oxide and related compounds. Related US patents 3,980,779; 3,868,371; and 4,001,410 disclose the preparation of a group of these compounds and their use as anti-microbial agents, particularly by addition of these materials to livestock fodder. U.S. Patent Nos. 3,991,189 and 3,957,799 disclose derivatives of these compounds bearing substituents on the nitrogen of the 3-amino group. These compounds also have anti-microbial activity.

The present invention provides additional compounds which specifically radiosensitize hypoxic cells and which, furthermore, are directly cytotoxic to hypoxic cells both in vitro and in vivo. Therefore, administration of these compounds prior to or following radiation treatment of tumors selectively kills the hypoxic (tumor) cells which survive the radiation dose. Both the ability of these compounds to radiosensitize hypoxic cells and especially their ability to selectively kill hypoxic cells directly are unexpected properties of these compounds.

The invention also provides novel 1,2,4-benzotriazine oxides useful as radiosensitizers and/or selective cytotoxic agents; and methods of synthesizing the compounds.

In 35th Ann. Meeting of Radiation Res. Soc. and 7th. Ann. Meeting of North Am. Hyperthermia Group, Feb. 21-26, 1987, Atlanta, US, 1986, page 114, No. FD-4; M. Brown et al. Bioreductive cytotoxic activity of 3-amino substituted 1,2,4-benzotriazine-1,4-dioxide is mentioned.

Int. J. Radiation Oncology Biol. Phys., 16, (4), April 1989, pages 977-981, E.W. Zeman et al. studied 3-amino-1,2,4-benzotriazine-1,4-dioxide and analogues thereof with a view to better understanding its mechanism. Proceedings of the Am. Association for Cancer Res. 18th. Ann. Meeting, May 24-27, 1989, 30, March 1989, page 575, No. 2290, M. Tracy et al. discussed 3-amino-1,2,4-benzotriazine-1,4-dioxide and 1,2,4-benzotriazine-1,4-dioxide as bioreductive agents that exhibit highly selective killing of hypoxic mammalian cells in vitro and of murine tumors in vivo.

Biochemical Pharmacology, 35, no. 19, October 1, 1986, pages 3417-3420, K.R. Laderoute et al., discusses 3-amino-1,2,4-benzotriazine-1,4-dioxide as a hypoxic cell toxic agent and investigates the major reduction products of this compound.

Radiotherapy and Oncology, 12, (1988), pages 209-218, E.M. Zeman et al. relates to a further study of 3-amino-1,2,4-benzotriazine-1,4-dioxide in the selective killing of hypoxic mammalian cells in vitro.

Int. J. Radiation Oncology Biol. Phys., 12, (7), 1986, pages 1239-1242, E.M. Zeman et al. is a further study on 3-amino-1,2,4-benzotriazine-1,4-dioxide as a bioreductive agent with selective toxicity with hypoxic mammalian cells.

Tetrahedron, 38, (12), 1982, pages 1793-1796, R.H. Atallah et al. describes the preparation of 3-methyl-1,2,4-benzotriazineoxides.

US-A-4027022 is concerned with antimicrobial compositions containing certain benzo-1,2,4-triazines-1,4-dioxides substituted in the 3 position with a substituted amino group and optionally substituted in the benzo ring.

DE-A-2404375 describes 1,2,4-benzotriazine-1,4-dioxides substituted in the 3 position by -NH-CO- or =C= groupings for use for the control of microorganisms, as feed additives for animals of commercial value and for the protection of materials.

WO-A-8908647, published 21 September 1989, describes the use of certain 1,2,4-benzotriazine oxides as radiosensitizers and selective cytotoxic agents.

WO-A-8802366 describes the use of certain 1,2,4-benzotriazine oxides as radiosensitizers and selective cytotoxic agents.

US-A-3079390 describes N-substituted 3-amino-1,2,4-benzotriazine-1-oxides in which the 3-substituent is the residue of a primary or secondary amine which are stated to have an inhibitory effect on the heat, swelling, redness and granuloma formation characteristic of the inflammatory response to tissue injury.

### Disclosure of the Invention

The invention provides a valuable addition to the group of compounds currently available as selective radiosensitizers and selective cytotoxic agents for hypoxic tumor cells. Some of the compounds now newly shown to be useful in this regard are known compounds. Others are themselves novel.

According to one aspect of the invention there is provided use of a compound of the formula 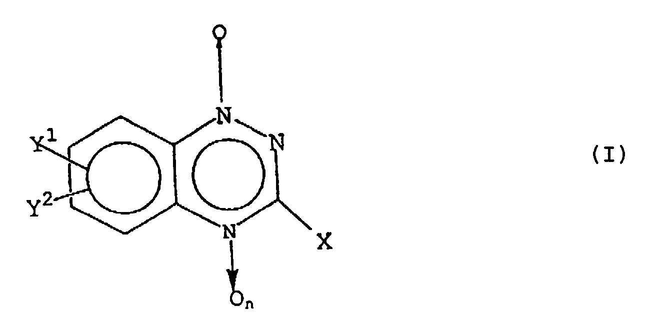 wherein
(a)
   X is NHR or NRR wherein each R is independently an alkyl of 1-4 carbon atoms substituted with alkyl (1-4C) secondary amino, dialkyl (1-4C) tertiary amino, morpholino, pyrrolidino or piperidino, or an acyl of 1-4 carbon atoms unsubstituted or substituted with OH, NH₂, alkyl (1-4C) secondary amino, dialkyl (1-4C) tertiary amino, morpholino, pyrrolidino, piperidino, alkoxy (1-4C), or halogen, or wherein in the case of NRR the two R groups may be linked together to form a morpholino, pyrrolidino or piperidino ring;
   n is 1; and
   Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl (1-4C) secondary amino, dialkyl (1-4C) tertiary amino, morpholino, pyrrolidino, piperidino, alkoxy (1-4C) or halogen substituents, preferably X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring, and more preferably, m is 1 or 2 and Y¹ and Y² are independently selected from the group consisting of H and nitro provided that one but not both of Y¹ and Y² are H,
   or
(b)
   X is NH₂, NHR or NRR wherein each R is independently an alkyl of 1-4 carbon atoms or acyl of 1-4 carbon atoms, or wherein in the case of NRR the two R groups may be linked together to form a morpholino, pyrrolidino or piperidino ring, and wherein R is unsubstituted or substituted with OH, NH₂, alkyl (1-4C) secondary amino, dialkyl (1-4C) tertiary amino, morpholino, pyrrolidino, piperidino, alkoxy (1-4C), or halogen, preferably X is NH₂
   n is 1; and
   Y¹ and Y² are chosen such that one but not both may be hydrogen, and one or both may be independently nitro; hydrocarbyl (1-14C) including cyclic and unsaturated hydrocarbyl, substituted with 1 or 2 substituents selected from the group consisting of pyrrolidino, piperidino, acetylaminoalkyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', O(SO)R', or O(POR')R' in which R' is a hydrocarbyl (1-4C) substituted with pyrrolidino or piperidino substituents, preferably Y' is H and Y² is nitro;
   or
(c)
   X is H or hydrocarbyl (1-4C);
   n is 1; and
   Y¹ and Y² are chosen such that one but not both may be H, and one or both may be independently nitro; hydrocarbyl (1-14C) including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or pyrrolidino, piperidino, NH₂, NHR', NR'R', O(CO)R', NH(CO)R', O(SO)R', or O(POR')R' in which R' is a hydrocarbyl (1-4C) which may be substituted with alkyl(1-4C) secondary amino, dialkyl (1-4C) tertiary amino, pyrrolidino or piperidino substituents with the proviso that when X is H, methyl or ethyl, and one of Y¹ and Y² is H, the other of Y¹ and Y² is not H;
   or
(d)
   X is hydrocarbyl (1-4C) substituted with OH, NH₂, alkoxy (1-4C), or halogen;
   n is 1; and
   Y¹ and Y³ are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C), and thio analogs thereof, acetyl aminoalkyl (1-4C), carboxy, alkoxycarbonyl (1-4C), carbamyl, alkylcarbamyl (1-4C), alkylsulfonyl (1-4C) or alkylphosphonyl (1-4C) wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents;
      or a pharmacologically acceptable salt thereof
      in the manufacture of a medicament for the treatment of the human or animal body for the selective killing of hypoxic tumor cells.

According to a second aspect of the invention, there is provided use of a compound of the formula 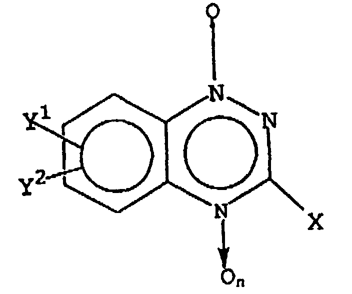 wherein
A.
   X is halogen; NHR or NRR, wherein the R groups are independently selected from alkyl (1-4C) substituted with lower alkyl (1-4C) secondary amino or dialkyl (1-4C) tertiary amino groups, acyl (1-4C) and acyl (1-4C) substituted with OH, NH₂, lower alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring;
   wherein n is 0 or 1; and
   Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents; preferably X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring, and more preferably m is 1 or 2 and Y¹ and Y² are independently selected from the group consisting of H and nitro;
   or
B.
   X is OH; alkoxy (1-4C); NH₂; NHR or NRR, wherein the R groups are independently alkyl (1-4C) or acyl-(1-4C) and are unsubstituted or substituted with OH, NH₂, lower alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring, preferably X is NH₂;
   n is 0 or 1; and
   Y¹ and Y² are chosen such that one but not both may be H, and one or both may be independently nitro; hydrocarbyl (1-14C), including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with pyrrolidino or piperidino substituents, preferably Y¹ is H, Y² is nitro and n is 1;
   or
C.
   X is hydrocarbyl (1-4C) substituted with OH, or NH₂; NHR or NRR, wherein the R groups are independently selected from substituted alkyl (1-4C) and substituted or unsubstituted acyl (1-4C), and the R groups are substituted with substituents selected from alkyl (1-4C) secondary and dialkyl (1-4C) tertiary amino groups, and in the case of NRR, the two R's can be linked together directly or through a bridge oxygen into a morpholino ring, pyrrolidino ring or piperidino ring;
   n is 0 or 1; and
   Y¹ and Y² are independently H; nitro; halogen, hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents;
   or
D.
   X is H; hydrocarbyl (1-4C); or hydrocarbyl (1-4C) substituted with OH, NH₂, NHR or NRR, wherein the R groups are independently selected from alkyl (1-4C) and acyl (1-4C), unsubstituted or substituted with OH, NH₂, alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring;
   n is 0 or 1; and
   Y¹ and Y² are chosen such that one but not both may be H, and one or both may be independently H; nitro; hydrocarbyl (1-14C), including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino piperidino, NH₂, NHR', NR'R', O(CO)R', NH(CO)R', O(SO)R', or O(POR')R' in which R' is a hydrocarbyl (1-4C) substituted with pyrrolidino or piperidino substituents with the proviso that when n is 1, X is H, methyl or ethyl and one of Y¹ and Y² is H, the other of Y¹ and Y² is not NH₂;
      or a pharmacologically acceptable salt thereof
      in the manufacture of a medicament for the treatment of the human or animal body for the radiosensitizing of hypoxic tumor cells.

According to a third aspect, the present invention provides a pharmaceutical composition comprising a compound of the formula 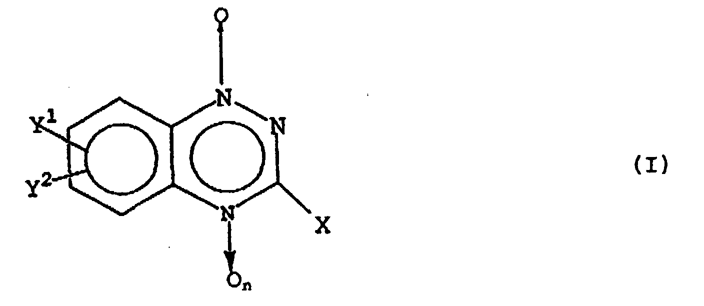 wherein
(i)
   X is NHR or NRR wherein each R is independently an alkyl of 1-4 carbon atoms substituted with alkyl (1-4C) secondary amino, dialkyl (1-4C) tertiary amino, morpholino, pyrrolidino or piperidino, or an acyl of 1-4 carbon atoms substituted with OH, NH₂, alkyl (1-4C) secondary amino, dialkyl (1-4C) tertiary amino, morpholino, pyrrolidino or piperidino, or wherein in the case of NRR the two R groups may be linked together to form a pyrrolidino ring;
   n is 1; and
   Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C) or halogen substituents, preferably X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring, and more preferably m is 1 or 2 and Y¹ and Y² are independently selected from the group consisting of H and nitro provided that one but not both of Y¹ and Y³ are H;
   or
(ii)
   X is NH₂, NHR or NRR wherein each R is independently an alkyl of 1-4 carbon atoms or acyl of 1-4 carbon atoms, or wherein in the case of NRR the two R groups may be linked together to form a morpholino, pyrrolidino or piperidino ring, and wherein R is unsubstituted or substituted with OH, NH₂, alkyl (1-4C) secondary amino, dialkyl (1-4C) tertiary amino, morpholino, pyrrolidino, piperidino, alkoxy (1-4C), or halogen, preferably X is NH₂;
   n is 1; and
   Y¹ and Y² are chosen such that one but not both may be hydrogen, and one or both may be independently nitro; hydrocarbyl (1-14C) including cyclic and unsaturated hydrocarbyl, substituted with 1 or 2 substituents selected from the group consisting of pyrrolidino, piperidino, acetylaminoalkyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', O(SO)R', or O(POR')R' in which R' is a hydrocarbyl (1-4C) substituted with pyrrolidino or piperidino substituents, preferably Y¹ is H and Y² is nitro;
   or
(iii)
   X is H or hydrocarbyl (1-4C);
   n is 1; and
   Y¹ and Y² are chosen such that one but not both may be H, and one or both may be independently nitro; hydrocarbyl (1-14C) including cyclic and unsaturated hydrocarbyl, optionally substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or pyrrolidino, piperidino, NH₂, NHR', NR'R', O(CO)R', NH(CO)R', O(SO)R', or O(POR')R' in which R' is a hydrocarbyl (1-4C) which may be substituted with alkyl-(1-4C) secondary amino, dialkyl (1-4C) tertiary amino, pyrrolidino or piperidino substituents with the proviso that when X is H, methyl or ethyl, and one of Y¹ and Y² is H, the other of Y¹ and Y² is not NH₂;
   or
(iv)
   X is hydrocarbyl (1-4C) substituted with OH, NH₂, alkoxy (1-4C), or halogen;
   n is 1; and
   Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino or piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C) wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents;
      or a pharmacologically acceptable salt thereof for use in a method of treatment of the human or animal body for the selective killing of hypoxic tumor cells.

According to another aspect of the invention, there is provided a pharmaceutical composition comprising a compound of the formula: 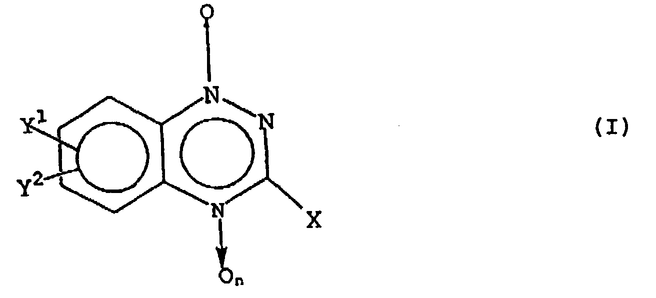 wherein
1.
   X is halogen; NHR or NRR, wherein the R groups are independently selected from alkyl (1-4C) substituted with lower alkyl (1-4C) secondary amino or dialkyl (1-4C) tertiary amino groups, and acyl (1-4C) substituted with OH, NH₂, or lower alkyl (1-4C) secondary or dialkyl (1-4C) tertiary amino groups, and in the case of NRR, the two R's can be linked together directly into a pyrrolidino ring;
   n is 0 or 1; and
   Y¹ and Y² are independently either H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents; preferably X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring, and more preferably m is 1 or 2 and Y¹ and Y² are independently selected from the group consisting of H and nitro;
   or
2.
   X is OH; alkoxy (1-4C); NH₂; NHR or NRR, wherein the R groups are independently alkyl (1-4C) or acyl (1-4C) and are unsubstituted or substituted with OH, NH₂, lower alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring, preferably X is NH₂;
   n is 0 or 1; and
   Y¹ and Y² are chosen such that one but not both may be H, and one or both may be independently H; nitro; hydrocarbyl (1-14C), including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with pyrrolidino or piperidino substituents, preferably Y¹ is H, Y² is nitro, and n is 1;
   or
3.
   X is hydrocarbyl (1-4C) substituted with OH, NH₂, NHR or NRR, wherein the R groups are independently selected from substituted alkyl (1-4C) and substituted acyl (1-4C), and the R groups are substituted with substituents selected from alkyl (1-4C) secondary and dialkyl (1-4C) tertiary amino groups, and in the case of NRR, the two R's can be linked together directly into a pyrrolidino ring;
   n is 0 or 1; and
   Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino piperidino, NH₂, NHR', NR'R', O(CO)R', NH(CO)R', O(SO)R' or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents;
   or
4.
   X is H; hydrocarbyl (1-4C); or hydrocarbyl (1-4C) substituted with OH, NH₂, NHR or NRR, wherein the R groups are independently selected from alkyl (1-4C) and acyl (1-4C), unsubstituted or substituted with OH, NH₂, alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring;
   n is 0 or 1; and
   Y¹ and Y² are chosen such that one but not both may be H, and one or both may be independently H; nitro; hydrocarbyl (1-14C), including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (O) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with pyrrolidino or piperidino substituents with the proviso that when n is 1, X is H, methyl or ethyl and one of Y¹ and Y² is not NH₂;
      or a pharmacologically acceptable salt thereof
      for use in a method of treatment of the human or animal body for the radiosensitizing of hypoxic tumor cells.

For use in fractionated radiotherapy according to the invention the cells requiring radiotherapy may be treated with a 1,2,4-benzotriazine oxide of Formula (I) before or after subjecting the treated cells to a plurality of distinct radiation doses over an extended period of time, each of the radiation doses being less than about 5 Gy.

The compounds useful in conjunction with the presently disclosed radiosensitizing methods are the mono- or dioxides of optionally substituted 1,2,4-benzotriazine which may contain a hydrocarbyl (1-4C), hydroxyl, alkoxy or amino group, either substituted or unsubstituted, in the 3-position, and their pharmacologically acceptable salts.

The compounds according to the invention for use in selectively killing hypoxic tumor cells are useful as selective cytotoxic agents are a subset of the above-defined compounds useful as radiosensitizers. That is, while all of the compounds defined by Formula (I) are generally effective as radiosensitizers, only those compounds unsubstituted at the 3-position or having a 3-amino or 3-hydrocarbyl (1-4C) substituent (i.e., X-H, hydrocarbyl (1-4C), NH₂, NHR or NRR with each R as defined above) and which are di-N-oxides (n-1) are effective cytotoxic agents. In this aspect of the invention, hypoxic tumor cells may be selectively killed by administering one or more of these compounds (or its salts) to the hypoxic tumor cells.

Certain of the compounds encompassed by Formula (I) are already known in the art as being useful for other purposes; other compounds are novel. The novel compounds encompassed by the present invention and which may be prepared by methods disclosed herein include compounds represented by Formula (I) (and their pharmacologically acceptable salts), in which the substituents fall into the following five classes:
I.
   X is NHR or NRR wherein each R is independently a substituted alkyl of 1-4 carbon atoms, or substituted acyl of 1-4 carbon atoms, or where the two R groups are alkyls linked together to form a pyrrolidino or piperidino ring or linked through an oxygen to form a morpholino ring, and the R groups are substituted with alkyl (1-4C) secondary amino, dialkyl (1-4C) tertiary amino, pyrrolidino or piperidino substituents;
   n is 1; and
   Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl (1-4C) secondary amino, dialkyl (1-4C) tertiary amino, morpholino, pyrrolidino, piperidino, alkoxy (1-4C), or halogen substituents, preferably Y¹ and Y² are both H, also preferably X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring, with m preferably being 1 or 2 and Y¹ and Y² independently selected from the group consisting of H and nitro;
II.
   X is OH, alkoxy (1-4C), NHR or NRR where each R is independently an alkyl of 1-4 carbon atoms, or acyl of 1-4 carbon atoms, or where the two R groups are alkyls linked together to form a pyrrolidino or piperidino ring or linked through an oxygen to form a morpholino ring, and the R groups may be further substituted with OH, NH₂, alkyl (1-4C) secondary amino, dialkyl (1-4C) tertiary amino, pyrrolidino, piperidino, alkoxy (1-4C), or halogen substituents, preferably X is OH or alkoxy or X is NRR;
   n is 1; and
   Y¹ and Y² are chosen such that one but not both may be hydrogen and one or both independently may be nitro; hydrocarbyl (1-14C), including cyclic and unsaturated hydrocarbyl, said hydrocarbyl being substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with pyrrolidino or piperidino substituents;
III.
   X is NH₂;
   n is 1; and
   Y¹ and Y² are chosen such that one but not both may be hydrogen, and one or both may be independently nitro, or saturated or unsaturated hydrocarbyl of 7-14C, or unsaturated hydrocarbyl of 2-6C, said hydrocarbyl groups being substituted with 1 or 2 substituents selected from the group consisting of lower alkyl (1-4C) secondary amino, dialkyl (1-4C) tertiary amino, pyrrolidino, piperidino, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with one or more alkyl (1-4C) secondary amino, dialkyl (1-4C) tertiary amino, pyrrolidino or piperidino substituents, preferably Y¹ is H and Y² is saturated or unsaturated hydrocarbyl of 7-14C, Y¹ is H and Y² is unsaturated hydrocarbyl of 2-6C, or Y¹ is H and Y² is nitro,
IV.
   X is hydrocarbyl (2-4C) substituted with OH, NH₂, alkoxy (1-4C) or halogen substituents;
   n is 1; and
   Y¹ and Y² are chosen such that one but not both may be hydrogen, and one or both may be independently nitro, or saturated or unsaturated by hydrocarbyl of 7 to 14C, or unsaturated hydrocarbyl of 2 to 6C, said hydrocarbyl groups being substituted with 1 or 2 substituents selected from the group consisting of alkyl (1-4C) secondary amino, dialkyl (1-4C) tertiary amino, pyrrolidino, piperidino, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with one or more, alkyl (1-4C) secondary amino, dialkyl (1-4C) tertiary amino, pyrrolidino or piperidino, substituents.
V.
   X is hydrogen or hydrocarbyl (2-4C);
   n is 1; and
   Y¹ and Y² are chosen such that one but not both may be hydrogen and both may be independently nitro; hydrocarbyl (1-14C), including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which may be substituted with one or more pyrrolidino or piperidino substituents with the proviso that when X is H or ethyl and one of Y¹ and Y² is H, the other of Y¹ and Y² is not NH₂;
      or a pharmacologically acceptable salt thereof.

A straightforward, one-step synthesis for preparing 1,2,4-benzotriazine oxides unsubstituted at the 3-position (i.e., the compounds of Formula (I) wherein X=H) comprises treating the corresponding 3-amino-1,2,4-benzotriazine oxide with a lower alkyl nitrite under reductive deaminating conditions. Accordingly there is provided a method of synthesizing a 1,2,4-benzotriazine oxide having the structure 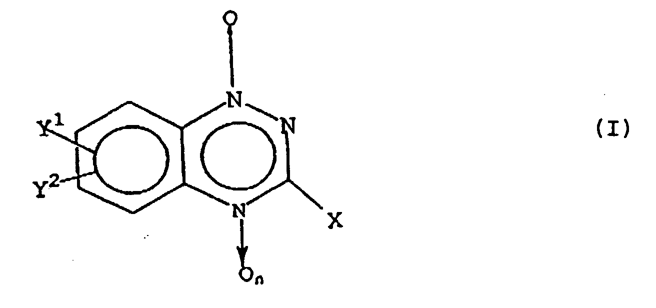 wherein
X is H; n is 1; and Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with one or more OH, NH₂, alkyl-(1-4C) secondary amino, dialkyl (1-4C) tertiary amino, morpholino, pyrrolidino, piperidino, alkoxy (1-4C), or halogen substituents,
or a pharmacologically acceptable salt thereof,
said method comprising:
treating a 3-amino-1,2,4-benzotriazine oxide having the structure 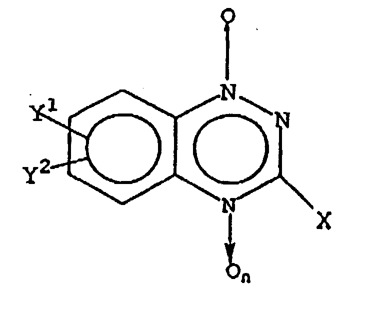 wherein
X is NH₂ with a lower alkyl nitrite preferably t-butyl nitrite in a compatible solvent, such as dimethylformamide, at a temperature of at least 60°C.

### Modes of Carrying Out the Invention

### A. The Compounds Useful in the Invention

The compounds useful as radiosensitizers and selective cytotoxic agents as described herein are derivatives of 1,2,4-benzotriazine oxide represented by Formula (I).

These compounds, as shown in the Formula (I), contain a group X in their 3 position. X will vary specifically as set forth above, depending upon the activity desired. Subject to the above-recited selection criteria, X is chosen in general from hydrogen; unsubstituted hydrocarbyls (1-4C) such as methyl, ethyl, s-butyl and the like; hydroxy; alkoxy (1-4C) such as methoxy, ethoxy, propoxy, t-butoxy and the like; primary amino (NH₂); secondary amino (NHR) where R is an alkyl or acyl of 1 to 4 carbons, such as methylamino, ethylamino and the like; tertiary amino (NRR) where each of the R groups is an alkyl or acyl of 1 to 4 carbons, for example diethylamino and the like, or the two R's join to form a morpholino, pyrrolidino or piperidino ring. In the case of the various alkyl and acyl R groups, they can be further substituted with OH, NH₂, lower alkyl (1-4C) secondary amino and dialkyl (1-4C) tertiary amino, morpholino pyrrolidino, piperidino, alkoxy (1-4C) or halogen (fluoro, chloro, bromo or iodo) substituents.

In the case of the hydrocarbyl X groups, they can be further substituted with OH, NH₂, alkyl secondary amino, dialkyl tertiary amino, alkoxy (1-4C) or halogen (fluoro, chloro, bromo or iodo) substituents.

The compounds of Formula (I) additionally contain groups Y¹ and Y². These groups are selected specifically according to the criteria set forth above, depending upon the utility desired.

Subject to these criteria, Y¹ and Y² may be selected from hydrogen; nitro; halogen (e.g. fluoro, chloro, bromo or iodo); or hydrocarbyl (1-14C). When hydrocarbyl, Y¹ or Y² may be saturated or unsaturated, cyclic or acyclic, and may optionally be interrupted by a single ether linkage. Thus, the unsubstituted hydrocarbyl forms of Y¹ or Y² can be, for example, methyl, ethyl, n-propyl, s-butyl, n-hexyl, 2-methyl-n-pentyl, 2-ethoxyethyl, 3-(n-propoxy)-n-propyl, 4-methoxybutyl, cyclohexyl, tetrahydrofurfuryl, furfuryl, cyclohexenyl, 3-(n-decyloxy)-n-propyl, 4-methyloctyl, 4,7-dimethyloctyl, or the like.

The hydrocarbyl Y¹ and Y² groups may optionally be substituted with 1 or 2 substituents selected from halogen such as fluoro, chloro, bromo or iodo; hydroxy; epoxy; alkoxy (1-4C) such as, for example, methoxy, n-propoxy and t-butoxy; alkyl thio; (1-4C) primary amino (NH₂); morpholino; pyrrolidino; piperidino; secondary amino (NHR') where R' is a 1-4C alkyl, such as methylamino, propylamino and the like; tertiary amino (NR'R'); acyloxy and acylamido groups represented by R'COO- and R'CONH-, respectively, and their thiol analogs represented by R'CSO- and R'CSNH-respectively; carboxy (-C(O)OH); alkoxycarbonyl (-C(O)OR'); carbamyl (-C(O)NH₂); alkylcarbamyl (1-4C) (-C(O)NHR¹); alkylsulfonyl (1-4C) (R'SO₂-); and alkyl phosphonyl (1-4C) ( R'P(OR')O-).

In addition Y¹ and Y² can each independently be -NH₂, -NHR', -NR'R', -OCOR', -NH(CO)R', -O(SO)R' or -O(POR')R' in which the various R' groups are lower alkyls (1-4C) which themselves may be substituted with OH, NH₂, alkyl secondary and tertiary amino, pyrrolidino, piperidino, alkoxy (1-4C), or halogen substitutents.

A particularly promising class of compounds for use both as radiosensitizers and selective cytotoxic agents include those represented by the following structural Formula (II): 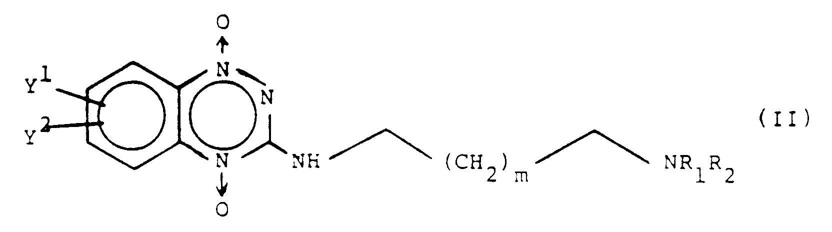 In Formula (II), one of Y¹ and Y² is H, the other an electron-withdrawing group (e.g., nitro, carboxy, alkoxycarbonyl, alkylsulfonyl), R₁ and R₂ are independently selected from the group consisting of hydrogen and lover alkyl, or the R₁ and R₂ groups may be linked to form a piperidino or pyrrolidino ring, and m is an integer from 0-4 inclusive, preferably 1 or 2.

Where X is OH, of course, the compounds may also be prepared and used as the pharmaceutically acceptable salts formed from inorganic bases, such as sodium, potassium, or calcium hydroxide, or from organic bases, such as caffeine, ethylamine, and lysine.

When X is NH₂, NHR, or NRR, e.g., NH-CH₂-(CH₂)ₘ-CH₂NR₁R₂ as in Formula (II), pharmaceutically acceptable acid addition salts may be used. These salts are those with inorganic acids such as hydrochloric, hydrobromic or phosphoric acids or organic acids such as acetic acid, pyruvic acid, succinic acid, mandelic acid, p-toluene sulfonic acid, and so forth. (Amino substituents on the hydrocarbyl side chain can also, of course, be converted to salts.)

The 1,2,4-benzotriazines may be used in the practice of this invention as the mono- or dioxides; i.e. either the 1-nitrogen of the triazino ring may be oxidized, or both the 1-and 4-nitrogens may be oxidized.

Specific particularly preferred compounds which are useful in the radiosensitization and cytotoxic procedures of the invention include
6(7)-nitro-3-amino-1,2,4-benzotriazine 1,4-dioxide;
3-(3-N,N-diethylaminopropylamino)-1,2,4-benzotriazine 1,4-dioxide;
6(7)-nitro-3-(2-N,N-diethylaminoethylamino)-1,2,4-benzotriazine 1,4-dioxide
   and their pharmaceutically acceptable salts and the thioamide analogs of the foregoing list of compounds. It should be noted that the Y¹ or Y² substituents set forth in the above compounds as present in either the 6 or 7 positions (designated "6(7)") may also be present at the 5 and/or 8 ring positions.

### B. Preparation of the Compounds of the Invention

General methods for preparing some 3-amino derivatives are found in the above-referenced patents to Ley et al., for example US 3,980,779. The compounds are prepared from benzofuroxan of the formula: 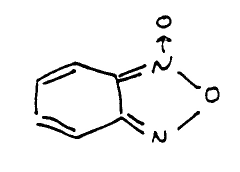 by reaction with a salt of cyanamide, followed by acidification of the reaction mixture. The benzofuroxan starting material is not symmetric with respect to its own 5 and 6 positions (which are the 6 and 7 positions of the resulting 3-amino benzotriazine oxide). Therefore, a mixture of the 6- and 7-substituted materials may result. If desired, this mixture can be separated using conventional means into individual components having a substituent in either the 6 or 7 position.

The dioxide may also be prepared from the parent monoxide or 1,2,4-benzotriazine by peracid oxidation (see Robbins et al, J Chem Soc 3186 (1957) and Mason et al, J Chem Soc B 911 (1970)).

In addition, the monoxide may be prepared by:
(1) cyclization of a 1-nitro-2-aminobenzene compound using H₂NCN·2HCl;
(2) oxidation of the parent compound given by the structure 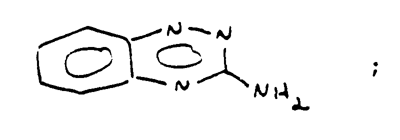 or by controlled reduction of the corresponding dioxide (see Mason, supra, and Wolf et al, J Am Chem Soc 76:355 (1954)).

The 1,2,4-benzotriazines may be prepared by cyclization of formazan precursors using BF₃/AcOH (see Scheme I and Atallah and Nazer, Tetrahedron 38:1793 (1982)).

3-amino-1,2,4-benzotriazines may be prepared either by cyclization of a parent compound (see Scheme II and Arndt, Chem. Ber. 3522 (1913)) or by reduction of the monoxide or dioxide as above.

The 3-hydroxy-1,2,4-benzotriazine oxides may be prepared using peroxide and sodium tungstate (Scheme III), a novel synthetic procedure for making the 3-hydroxy-1,4-dioxide compound, or concentrated sulfuric acid and sodium nitrate (Scheme IV).

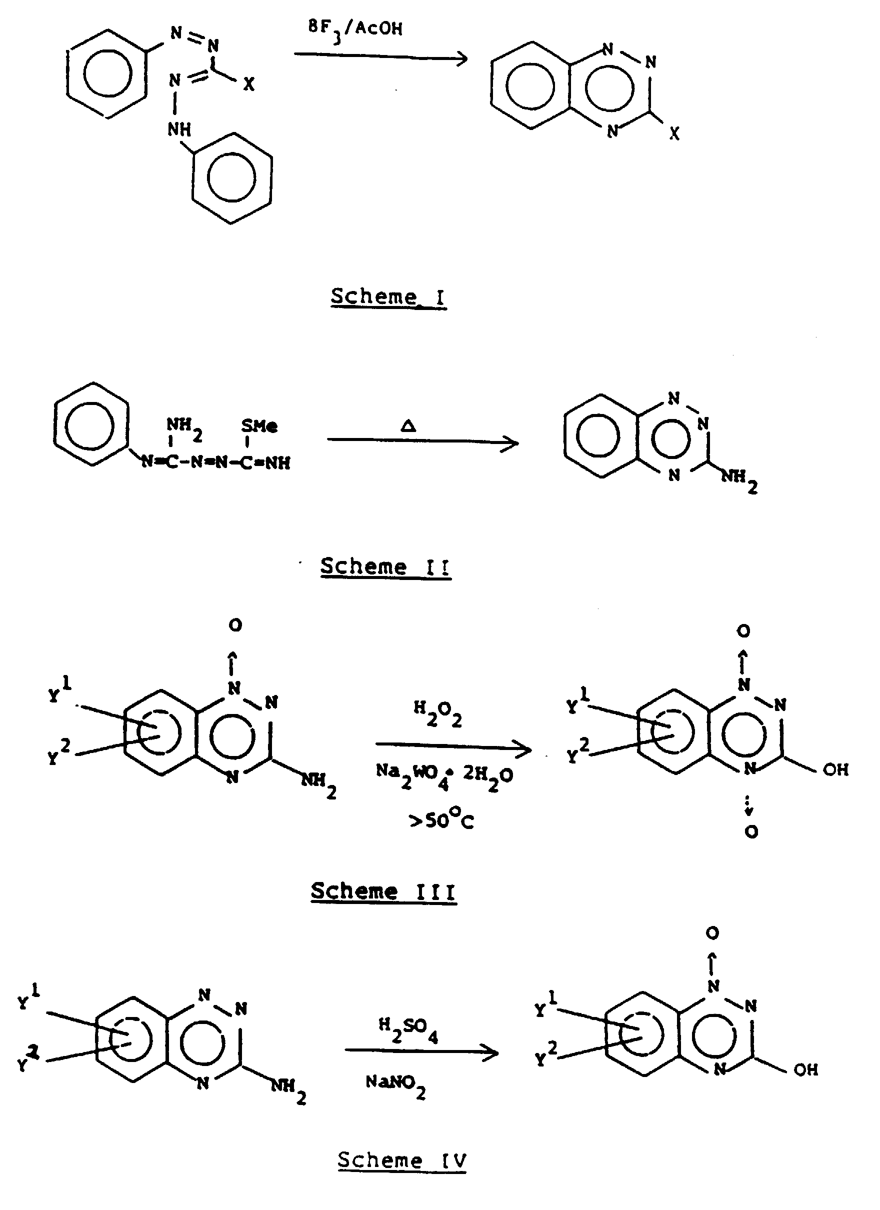

The invention also encompasses a novel method of preparing 1,2,4-benzotriazine oxides unsubstituted at the 3 position (sometimes referred to herein as the "3-desamino" compounds). The novel synthesis involves reductive deamination of the corresponding 3-amino structure. In contrast to prior methods of synthesizing 3-desamino-1,2,4-benzotriazine oxides, the present method enables a simple, straightforward one-step method which gives the desired product in a high yield. The method involves treating a 1,2,4-benzotriazine oxide of Formula (I), wherein X is NH₂, with a lower alkyl nitrite under reductive deaminating conditions. By "reductive deaminating conditions" is meant reaction conditions which will give rise to at least about 10%, preferably at least about 50%, of the desired 3-unsubstituted reaction product. A preferred lower alkyl nitrite for use in said method is t-butyl nitrite. Exemplary reductive deaminating conditions involve reaction in a compatible solvent, e.g., dimethyl formamide, at a temperature of at least about 60°C, typically at a temperature in the range of 60-65°C. This reaction is illustrated generally at Scheme V, and is exemplified in Examples 4-7 herein.

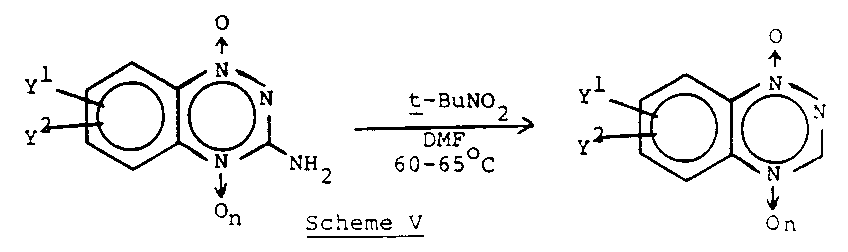

### C. Formulation and Administration

As demonstrated below, the oxidized benzotriazines of the invention may be used to radiosensitize or selectively kill hypoxic tumor cells in warm-blooded animal hosts. A way in which they may be used is in conjunction with agents known to selectively create hypoxia in tumors. Such methods include the use of antihypertensive drugs such as hydralazine, or agents which affect the amount of oxygen carried by the blood. While these compounds will typically be used in cancer therapy of human patients, they may be used to kill hypoxic tumor cells in other warm blooded animal species such as other primates, farm animals such as cattle, and sports animals and pets such as horses, dogs, and cats.

Hypoxia is believed to be associated with all types of solid malignant neoplasms. The compounds of the invention may, therefore, be used to radiosensitize or to kill neoplastic epithelial cells, endothelial cells, connective tissue cells, bone cells, muscle cells, nerve cells, and brain cells. Examples of carcinomas and sarcomas include carcinomas such as epithelial cell, acidic cell, alveolar cell, basal cell, basal squamous cell, cervical, renal, liver, Hurthle, Lucke, mucinous and Walker, and sarcomas such as Abernathy's, alveolar soft part, angiolithic, botyroid, encephaloid, endometria stroma, Ewing's fascicular, giant cell, lymphatic, Jensen's, juxtacortical osteogenic, Kaposi's, medullary, and synovial. Specific examples of tumors that have been sensitized with other radiosensitizers are reported in Adams, G.E., Cancer: A Comprehensive Treatise (F. Becker, Ed) vol 6, pp 181-223, Plenum, New York, 1977.

The compounds may be administered to patients orally or parenterally (intravenously, subcutaneously, intramuscularly, intraspinally, intraperitoneally, and the like). When administered parenterally the compounds will normally be formulated in a unit dosage injectable form (solution, suspension, emulsion) with a pharmaceutically acceptable vehicle. Such vehicles are typically nontoxic and nontherapeutic. Examples of such vehicles are water, aqueous vehicles such as saline, Ringer's solution, dextrose solution, and Hank's solution and nonaqueous vehicles such as fixed oils (e.g., corn, cottonseed, peanut, and sesame), ethyl oleate, and isopropyl myristate. Sterile saline is a preferred vehicle and the compounds are sufficiently water soluble to provide a solution for all foreseeable needs. The vehicle may contain minor amounts of additives such as substances that enhance solubility, isotonicity, and chemical stability, e.g., antioxidants, buffers, and preservatives. When administered orally (or rectally) the compounds will usually be formulated into a unit dosage form such as a tablet, capsule, suppository or cachet. Such formulations typically include a solid, semisolid or liquid carrier or diluent. Exemplary diluents and vehicles are lactose, dextrose, sucrose, sorbitol, mannitol, starches, gum acacia, calcium phosphate, mineral oil, cocoa butter, oil of theobroma, alginates, tragacanth, gelatin, methylcellulose, polyoxyethylene sorbitan monolaurate, methyl hydroxybenzoate, propyl hydroxybenzoate, talc, and magnesium stearate.

The amount of compound administered to the subject is sufficient to radiosensitize or to produce cytotoxicity in the malignant neoplasm to be treated but below that which may elicit toxic effects to normal tissue. This amount will depend upon the type of tumor, the species of the subject being treated, the indicated dosage intended and the weight or body surface of the subject. The radiation may be administered to humans in a variety of different fractionation regimes, i.e., the total radiation dose is given in portions over a period of several days to several weeks. These are most likely to vary from daily (i.e., five times per week) doses for up to six weeks, to once weekly doses for four to six weeks. An individual dose of the benzotriazine will be given before or after each radiation treatment and is likely to be in the range of 0.01 to 20 mmol/kg and usually in the range of 0.1 to 2 mmol/kg. In these treatment regimens, each radiation dose is typically 1-5 Gy, preferably, less than 2.5 Gy and more preferably 2-2.5Gy. Typically, one dose of radiation is administered per day although 2 or more might be used if tolerated by the patient

It has now been found that the compounds disclosed herein as radiosensitizers both sensitize tumors to radiation without increasing the sensitivity of normal skin, and work in highly fractionated radiation regimens.

For use as selective cytotoxic agents, the compounds of the invention can be administered alone, with radiation or other cancer cytotoxic agents, with vasoactive drugs (e.g., hydralazine), or under other conditions which reduce the amount of available oxygen carried by the blood such as anemia or drugs which increase the binding of oxygen to hemoglobin, all of which can enhance selectively the degree of hypoxia in the tumor.

### Examples

The following examples further illustrate the compounds of the invention and methods for synthesizing and using them, and are not intended to limit the invention in any manner.

Experimental: All reactions were carried out in flame-dried glassware and under a blanket of Argon. t-butyl nitrite (90%) was purchased from the Aldrich Chemical company. Dimethylformamide was distilled from calcium hydride. 7-Nitro-3-amino-1,2,4-benzotriazine-3-amine 1-oxide was purchased from Parish Chemical Company, trifluoroacetic anhydride, N,N-diethylethylenediamine, N,N-diethylpropylenediamine and sodium tungstate dihydrate were purchased from Aldrich Chemical Company and 70% hydrogen peroxide was a gift from Interox America. All reactants were used without further purification. Flash chromatography was carried out on E. Merck 230-400 mesh silica gel under a positive pressure of argon. NMR spectra were obtained on a Varian XL-400 or Jeol FX90Q spectrometer and in d₆-acetone, d₄-methanol, or d₆-dimethyl sulfoxide, as indicated, and are reported relative to the central peak in the appropriate multiplet (2.04, 3.30, and 2.49 ppm, respectively), UV spectra were obtained on a Perkin-Elmer 552 spectrophotometer in 95% ethanol, mass spectra were obtained on an LKB 9000 mass spectrometer, and elemental analyses were carried out by Desert Analytics, Tucson, AZ.

### Example 1: Preparation of 7-Nitro-3-Amino-1,2,4-Benzotriazine 1,4-Dioxide

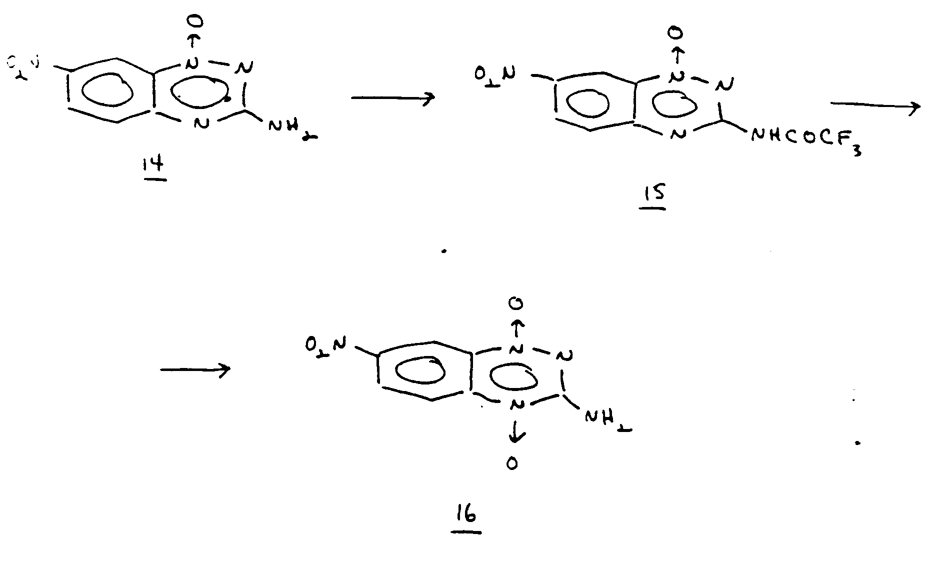

7-Nitro-3-trifluoroacetamido-1,2,4-benzotriazine 1-oxide (15): A solution of 7-nitro-3-amino-1,2,4-benzotriazine 1-oxide (14) (4.00g, 19.3 mmol; Parish Chemical Co.), CHCl₃ (125 ml) and trifluoroacetic anhydride (12.0 ml, 85.0 mmol) was stirred at room temperature for 44 hr. The resultant light yellow solid was filtered, washed with CHCl₃ (50 ml) and dried to give 5.35g (91% yield) of the product as a yellow solid. Anal. Calc'd. for C₉H₄F₃N₅O₄: C, 35.7; H, 1.33; N, 23.10. Found: C, 35.7; H, 1.23; N, 23.06.

7-Nitro-3-amino-1,2,4-benzotriazine 1,4-oxide (16): To a stirred solution of 7-nitro-3-trifluoroacetamido-1,2,4-benzotriazine 1-oxide prepared above (15) (2.509, 8.25 mmol) in CHCl₃ (200 ml) was added Na₂WO₂.2H₂O (90 mg, 0.273 mmol) followed by 70% H₂O₂ (10 ml). After 15 min the solution was treated with trifluoroacetic anhydride (8.0 ml, 56.7 mmol) and stirring was continued at room temperature for 64 hr. The reaction mixture was chromatographed (EtOAc, 20% MeOH/acetone, and finally 20% DMF/acetone) then recrystallized in acetone to give 1.20g (65% yield) of the product (16) as an orange solid, mp 286-288°C (dec.). UV: λ 259, 300, 345, 387, 472. Anal. Calc'd. for C₇H₅N₅O₄: C, 37.70; H, 2.26; N, 31.39. Found: C, 37.70; H, 2.13; N, 30.94.

### Example 2: Preparation of 3-(3-N,N-Diethylaminopropylamino)-1,2,4-Benzotriazine 1,4-Dioxide

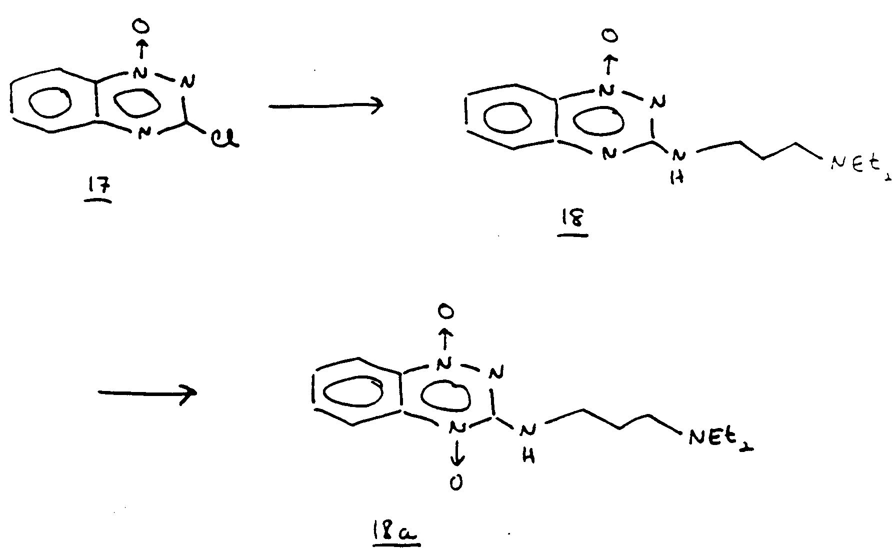

3-(3-N,N-diethylaminopropylamino)-1,2,4-benzotriazine 1-oxide (18): A solution of 3-chloro-1,2,4-benzotriazine 1-oxide (17) (3.0g, 16.5 mmol) (produced by the method of Sasse et al., U.S. Patent 4,289,771) in CH₂Cl₂ (100 ml) was treated with N,N-diethylpropylenediamine (9.5 ml, 88.3 mmol). After 20 hr at room temperature the mixture was diluted with 1,2-dichloroethane (50 ml) and washed successively with Na₂CO₃ and H₂O. The yellow solution was dried (Na₂SO₄), filtered and evaporated in vacuo to give 3.93g (87% yield) of the product as a yellow solid. Recrystallization (ether/petroleum ether) yielded pure material, mp 47-48°C. Anal Calc'd. for C₁₄H₂₁N₅O (18): C, 61.10; H, 7.69; N, 25.44. Found: C, 61.30; H, 7.80; N, 25.61.

3-(3-N,N-diethylaminopropylamino)-1,2,4 benzotriazine 1,4-dioxide (18a): To a stirred solution of 3-(3-N,N-diethylaminopropylamino)-1,2,4-benzotriazine 1-oxide 18 prepared as above (1.60g, 6.10 mmol) in CHCl₃ (50 ml) was added trifluoroacetic anhydride (22.0 ml). After 15 min the mixture was cooled to -10°C, 70% H₂O₂ (10 ml) added and then stirred at room temperature for 20 days. The reaction mixture was dried (Na₂SO₄), filtered and evaporated to dryness. The residue was dissolved in saturated NaHCO₃ solution (50 ml) and extracted with CH₂Cl₂ (3x150 ml). The organic layer was dried (Na₂SO₄), filtered and evaporated to give the product 18a, 0.51g (29% yield) as a red solid, mp 92-94°C. NMR: δ (400 MHz, CDCl₃) 1.11 (6H, t, J=7.1 Hz, CH₃), 1.84-1.90 (2H, m, H-2'), 2.48-2.64 (4H, m, NCH₂6H₃ and H-3'), 3.68 (2H, br t, J=5.5 Hz, H-1'), 7.46 (1H, ddd, J=7.1, 7.0 and 1.2 Hz, H-6), 7.84, ddd, J=7.0, 6.9 and 1.2 Hz, H-7), 8.31 (2H, m, H-5 and 8), 8.80 (1H, br s, NH). UV: λ 220, 270, 476. Anal. Calc'd. for C₁₄H₂₁N₅O₂. (1/3 H₂O): C, 56.50; H, 7.34; N, 23.55. Found: C, 56.90; H, 7.15; N, 23.40.

### Example 3: Preparation of 7-Nitro-3-(2-N,N-Diethylaminoethylamino)-1,2,4-Benzotriazine 1,4-Dioxide

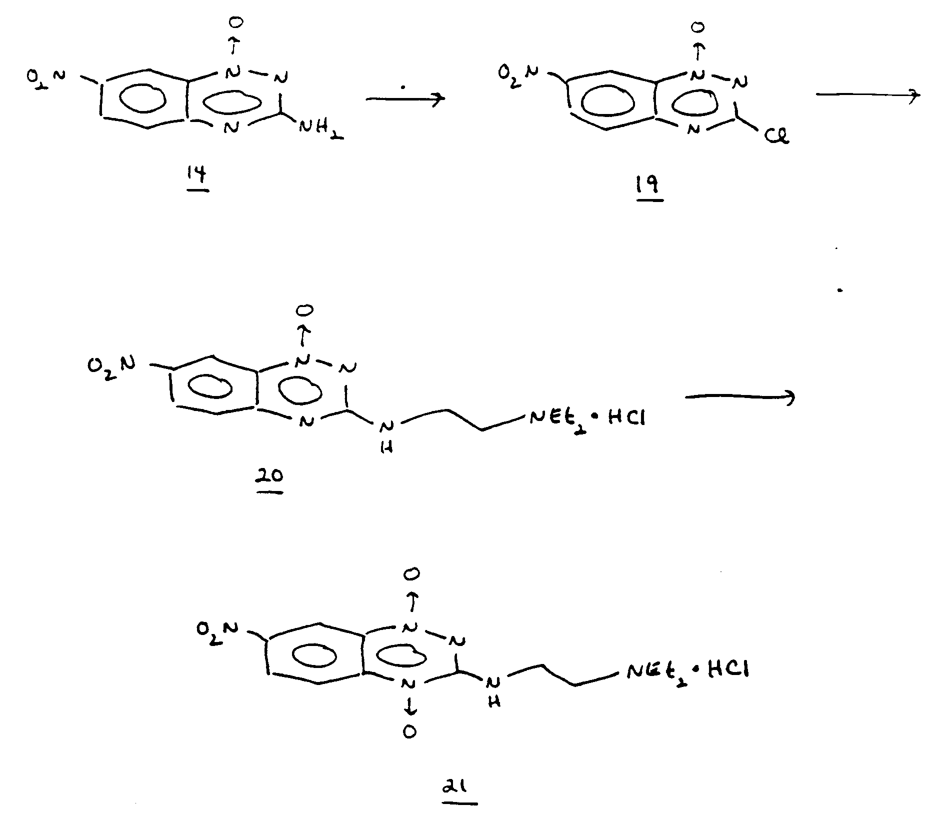

7-nitro-3-(2-N,N-diethylaminoethylamino)-1,2,4-benzotriazine 1-oxide hydrochloride (20): A solution of 7-nitro-3-chloro-1,2,4-benzotriazine 1-oxide (19) (1.60g, 7.06 mmol) (prepared as generally shown in Sasse et al., U.S. Patent 4,289,771, with (a) NaNO₂ and H₂SO₄ at 40°C, followed by (b) chlorination with POCl₃ at 106°C) in CH₂Cl₂ (50 ml) was treated with N,N-diethylethylenediamine (6.0 ml, 42.7 mmol). After 16 hr at room temperature the mixture was evaporated to dryness under high vacuum at 60°C. The yellow solid was stirred in 20% iPrOH/ether (150 ml) for 5 hr, filtered, washed with iPrOH then petroleum ether and dried (80°C/1.0 mmHg) to give 1.80g (74% yield) of the product 20 as yellow needle crystals. NMR δ (90 MHz, d₆-DMSO/d₄-MeOH) 1.25 (6H, t, J=6.0 Hz, CH₃), 3.25 (6H, m, NCH₂), 3.82 (2H, m, H-1'), 7.74 (1H, d, J=7.0 Hz, H-5), 8.52 (1H, dd, J=7.0 and 2.0 Hz, H-6), 8.91 (1H, d, J=2.0 Hz, H-8).

7-nitro-3-(2-N,N-diethylaminoethylamino)-1,2,4-benzotriazine 1,4-dioxide hydrochloride (21). To a stirred solution of 7-nitro-3-(2-N,N-diethylamino-ethylamino)-1,2,4-benzotriazine 1-oxide hydrochloride (20; prepared as described above) (0.50g, 1.46 mmol) in CHCl₃ (50 ml) at 0°C was added trifluoroacetic anhydride (9.0 ml). After 30 min 70% H₂O₂ (4.0 ml) was added and the mixture stirred at room temperature for 3 days, then dried (Na₂SO₄), filtered, and evaporated in vacuo to dryness to give the trifluoroacetate salt 0.67g (45% yield). This product was dissolved in saturated NaHCO₃ solution (30 ml) and extracted with CH₂Cl₂ (3x30 ml). The dichloromethane was washed with H₂O, dried (Na₂SO₄), filtered, saturated with gaseous HCl and evaporated to dryness to give 0.35g (63% yield, 28% overall) of the product as a red solid, m.p. 194-195°C. UV: λ 260, 306, 388, 479. Anal. Calc'd. for C₁₃H₁₈N₆O₄.HCl: C, 43.50; H, 5.34; N, 23.43. Found: C, 43.20; H, 5 37; N, 23.11.

The following Examples 4-7 are directed to reductive deamination reactions for preparing compounds of Formula (I) which are unsubstituted at the 3-position, i.e., wherein the substituent "X" is hydrogen.

### Example 4: Preparation of 1,2,4-Benzotriazine 1,4-Dioxide by Reductive Deamination of 3-Amino-1,2,4-Benzotriazine 1,4-Dioxide

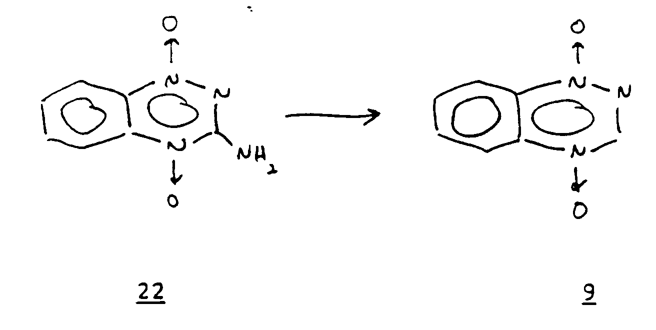

To a rapidly stirred solution of t-butyl nitrite (867 mg, 1.0 ml, 8.41 mmol) in DMF (20 ml) at 60-65°C was added 3-amino-1,2,4-benzotriazine 1,4-dioxide ("SR 4233") (500 mg, 2.81 mmol) (prepared by the method of Seng et al., Angew. Chem. Internat. Edit. 11:11 (1972)) in small portions over 5 min. Following the addition, and subsidence of the concomitant effervescence (approx. 5 min), the solution was cooled and reduced under high vacuum to a dark waxy solid. Flash chromatography (30% EtOAc/CH₂Cl₂) gave a yellow solid, mp 188-189.5°C (dec.), which was recrystallized from methanol to give 195 mg (43% yield) of the product 9 as bright yellow platelets, mp 192-194°C (dec.). NMR: δ (400 MHz, d₆-acetone) 8.04 (1 H, ddd, J=8.5, 7, 1.5 Hz), 8.15 (1 H, ddd, J=8.5, 7, 1.5 Hz), 8.42 (1 H, dd, J=8.5, 1.5 Hz), 8.43 (1 H, dd, J=8.5, 1.5 Hz) 9.05 (1 H, s, H-3). UV: λ 405, 300, 225. MS, m/z (relative intensity) 164(9), 163(100, M⁺), 147(13), 136(19), 90(7), 78(27), 76(26), 75(8), 64(9), 63(10), 52(12), 51(48), 50(28), 38(8), 37(5), 30(18), 28(6), 27(7). Anal. Calc'd. for C₇H₅N₃O₂: C, 51.54; H, 3.09; N, 25.76. Found: C, 51.42; H, 3.02; N, 25.66.

### Example 5: Preparation of 7-Allyloxy-1,2,4-Benzotriazine 1,4-Dioxide Via Reductive Deamination

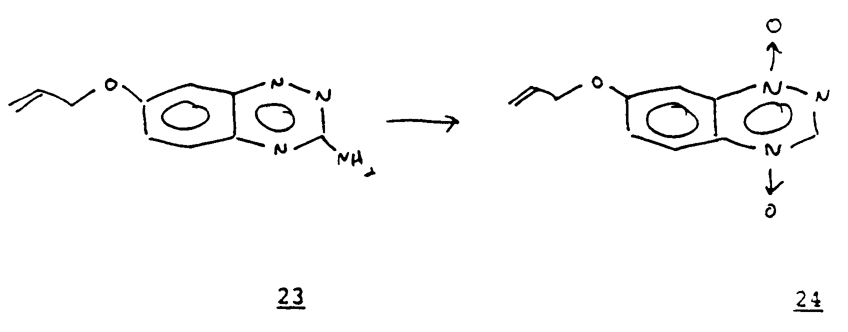

7-Allyloxy-1,2,4-benzotriazine 1,4-dioxide 24: To a stirred solution of t-butyl nitrite (271 mg, 0.312 ml, 2.63 mmol) in DMF (15 ml) at 60-65°C was added 7-allyloxy-3-amino-1,2,4-benzotriazine- 1,4-dioxide 23 (205 mg, 0.875 mmol) in small portions over 5 min. After 30 min additional t-butyl nitrite (271 mg, 0.312 ml, 2.63 mmol) was added, and shortly thereafter the deep red solution effervesced and lightened appreciably in color over a period of a few minutes. After an additional 30 min the resultant orange solution was reduced under vacuum to a brown solid which was sequentially flash chromatographed (10% EtOAc/CH₂Cl₂) and crystallized (CH₂Cl₂/petroleum ether) to give 72 mg (38% yield) of the product 24 as light orange crystals, mp 147-148°C. NMR: δ (400 MHz, d₆-acetone) 4.89 (2 H, ddd, H-1', J_{1',2'}=5.5, J_{1',3'cis}=J_{1',3'trans}=1.5 Hz), 5.36 (1 H, ddd, H-3', J_{3',2'cis}=10.5, J_{3',3'}=3, J_{3',1'}=1.5 Hz), 5.52 (1 H, ddd, H-3', J_{3',2'trans}=17.5, J_{3',3'}=3, J_{3'1'}=1.5 Hz), 6.14 (1 H, ddt, H-2', J_{2',3'cis}=10.5, J_{2',1'}=5.5 Hz), 7.70 (1 H, d, H-8, J_{8,6}=2.5 Hz), 7.74 (1 H, dd, H-6, J_{6,5}=9.5, J_{6,8=2.5} Hz), 8.33 (1 H, d, H-5, J_{5,6}= 9.5 Hz), 8.93 (1 H, s, H-3). UV: λ 425, 410, 365, 355, 320, 245, 200. MS m/z/ (relative intensity) 220(4), 219(34,M⁺), 103(4), 77(4), 75(4), 63(13), 62(4), 42(3), 41(100), 39(16). Anal. Calc'd. for C₁₀H₉N₃O₃: C, 54.79; H, 4.14; N, 19.17. Found: C, 54.73; H, 4.16; N. 19.15.

### Example 6: Preparation of 7-(3-N-Ethylacetamido-2-acetoxypropoxy)-1,2,4-Benzotriazine 1,4-Dioxide Via Reductive Amination

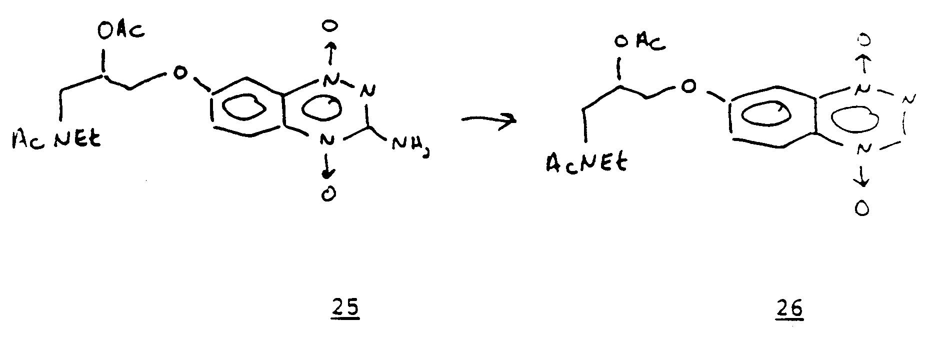

To a stirred solution of t-butyl nitrite (185 mg, 1.79 mmol) in DMF (5 ml) at 60°C was added via syringe a solution of 7-(3-N-ethylacetamido-2-acetoxypropoxy)-3-amino-1,2,4-benzotriazine 1,4-dioxide (25) (125 mg, 0.329 mmol) in DMF (5 ml) over a period of 1 min. After 5 min additional t-butyl nitrite (217 mg, 2.10 mmol) was added and an immediate reaction occurred, as evidenced by the evolution of a gas and a change in color of the solution from red to light orange. After an additional 10 min the solution was stripped to a yellow/brown solid and eluted through silica gel with 5% MeOH/CH₂Cl₂ to give 119 mg of a yellow oil. Recrystallization from CH₂Cl₂/ligroin gave 90 mg yellow solid (75% yield), mp 179-180.5°C. NMR: δ (400 MHz, d₄-methanol, mixture of rotamers, ratio approx. 2:1) 1.12, 1.22 (t's, 1:2, 3 H total, J=7 Hz), 2.06, 2.07 (s's, 2:1, 3 H total), 2.11, 2.17 (s's, 2:1, 3 H total), 3.41-3.92 (m, 4 H), 4.34-4.48 (m, 2 H), 5.48-5.58 (m, 1 H), 7.76-7.86 (m, 2 H), 8.36-8.42 (m, 1 H), 9.04, 9.06 (s's, 2:1, 1 H total). UV: λ 420, 405, 365, 350, 315, 240, 200. MS: m/z (relative intensity) 365(0.5), 364(1.4, M⁺), 349(0.5), 348(1.1), 347(0.5), 332(1.2), 331(3.6), 187(7), 186(66), 102(6), 100(21), 84(30), 63(6), 58(100), 56(8), 43(65), 42(9), 41(5), 30(14), 29(5), 28(8).

### Example 7: Preparation of 7-Nitro-1,2,4-Benzotriazine 1,4-Dioxide via Reductive Demination

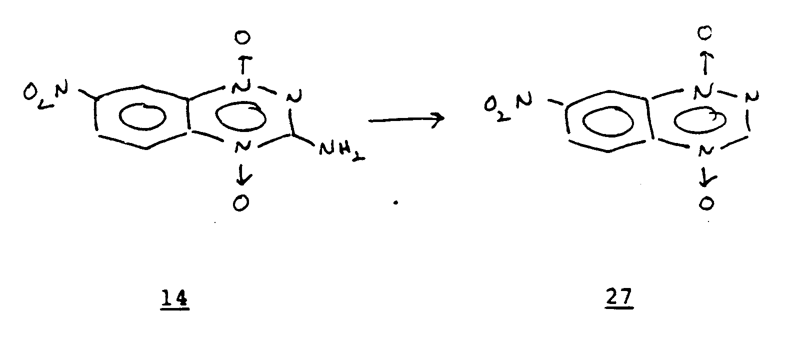

To a stirred solution of t-butyl nitrite (88 mg, 0.85 mmol) in DMF (5 ml) at 60°C was added 7-nitro-3-amino-1,2,4-benzotriazine 1,4-dioxide (14) (38 mg, 0.17 mmol). After 30 min the addition of further t-butyl nitrite (175 mg, 1.70 mmol) to the dark red slurry was immediately followed by a change in coloration and effervescence. After an additional 10 min the orange solution was reduced to a red solid in vacuo and chromatographed with 1% AcOH/CH₂Cl₂ to give 3 mg of the product 27 as a yellow solid (10% yield). NMR δ (90 MHz, d₆-dimethyl sulfoxide) 7.68 (d, 1H, J = 9.2 Hz), 7.92 (dd, 1H, J = 9.2, 2.2 Hz), 8.10 (d, 1H, J = 2.2 Hz), 8.65 (s, 1H)). UV: λ 420, 310, 240, 205. MS:m/z (relative intensity) 209 (9), 208 (100, M⁺), 192 (54), 181 (14), 162 (16), 105 (9), 77 (28), 75 (52), 74 (27), 63 (21), 62 (16), 30 (77), 18 (26).

### Example 8: Determination of LD₅₀

LD₅₀ is determined in BALB/c female mice (weighing 20-25 g) following intraperitoneal (ip) injection, unless the compound tested has low lipophilicity and is very soluble, wherein intravenous (iv) administration is used. LD₅₀ values at 1, 2, 5, and 60 days are determined by administering graded doses of the drug dissolved in physiological saline immediately prior to injection.

### Example 19: Radiosensitivity in Vitro

The results of assays to determine the concentration of drug necessary to produce a sensitizer enhancement ratio of 1.6 of hypoxic cells in culture are as follows:

### Example 9: Physicochemical and Biological Properties of Some 1,2,4-Benzotriazine 1,4-Dioxides

The following table sets forth various properties of compounds 22, 14, 18a, and 21 as determined by the inventors herein:

| Cpd. | Mol. Wt. | Solubility (mM) | Log P^{a} | E1/2^{b} (mV) | RHT^{c} | HCR^{d} | LD₅₀^{e} |
|---|---|---|---|---|---|---|---|
| 22 | 178.2 | 13.50 | -0.32 | -332 | 1 | 100 | 0.50 |
| 14 | 223.2 | 2.00 | -0.97 | -133 | 2 | 140 | 1.00 |
| 18a | 291.4 | >181 | -1.34 | -348 | 3 | 100 | 0.25 |
| 21 | 358.9 | 96.40 | -0.20 | -140 | 3 | 211 | 0.40 |

| | | | | | | | |
|---|---|---|---|---|---|---|---|
| a.) Log of the octanol-water partition coefficient as measured by the method of Fujita et al., J. Amer. Chem. Soc. 86:5175 (1964), using pH 7.4 buffer. | | | | | | | |
| b.) Polarographic half-wave reduction potentials measured in Britton & Robinson pH 7.4 buffer using a dropping mercury electrode. | | | | | | | |
| c.) Relative Hypoxic Cytoxicity: Ratio of equitoxic concentrations of 22:analog for HA-1 cells attached, under hypoxic conditions. Exponentially growing cells were placed in suspension culture and gassed for 90 min in nitrogen or air prior to addition of drugs. Samples were removed periodically for a survival determination, and the ratios determined from a comparison of the resulting survival curves. | | | | | | | |
| d.) Hypoxic Cytoxicity Ratio: Ratio of equitoxic concentrations of each analog for HA-1 cells attained under hypoxic:aerobic conditions. Treatment conditions as above. | | | | | | | |
| e.) Balb/c female mice 3-5 months of age were used in the LD₅₀ experiments. LD₅₀ was evaluated as described in Example 8. | | | | | | | |

As may be readily deduced from the table, novel compounds 14, 18a,and 21 exhibit significantly enhanced cytotoxicity against hypoxic HA-1 tumor cells in vitro compared to 3-amino-1,2,4-benzotriazine 1,4-dioxide (22), while retaining the high differential cytotoxicity against hypoxic cells compared to aerobic cells. These results suggest that these drugs will be more tumor-specific and therefore more effective as antitumor agents in vivo.

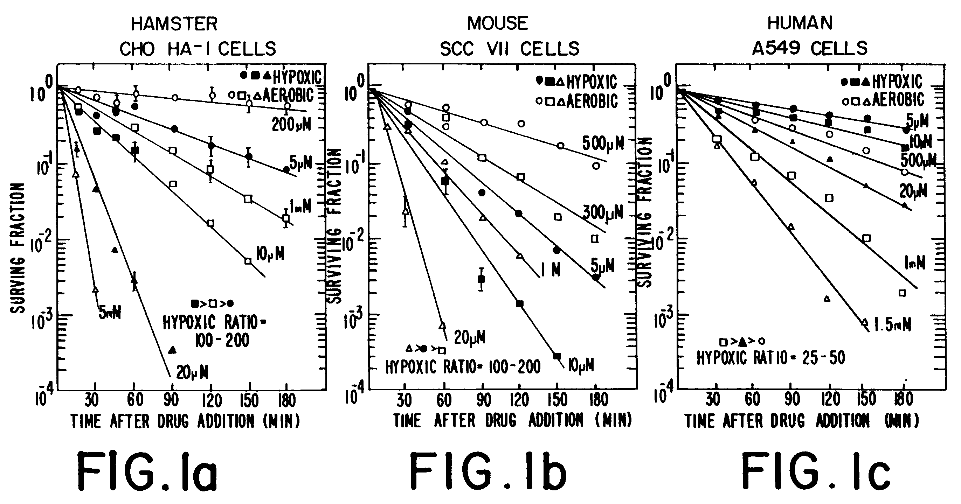

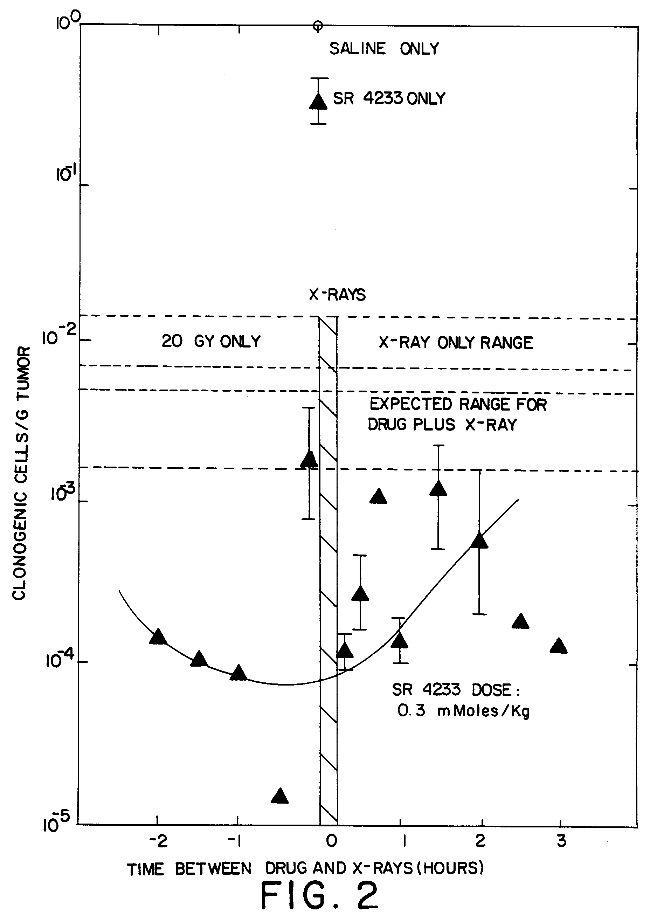

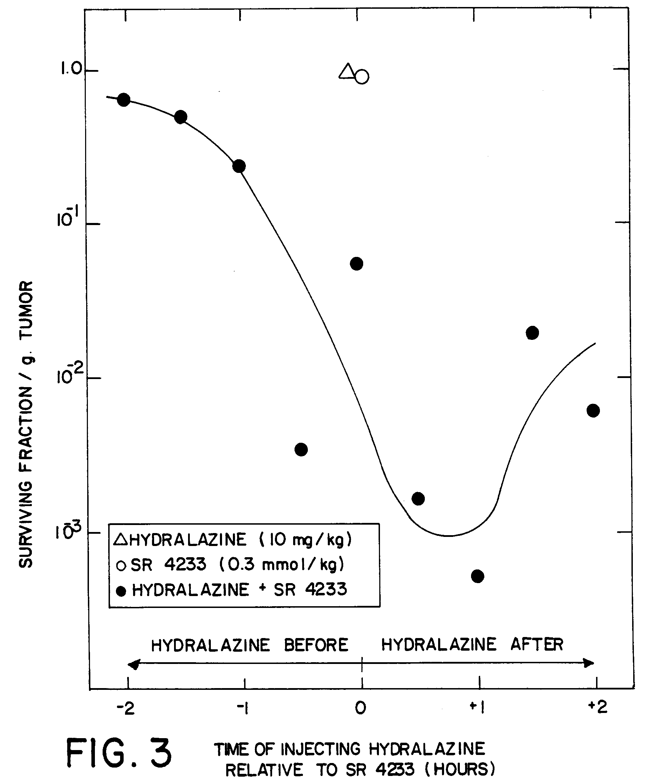

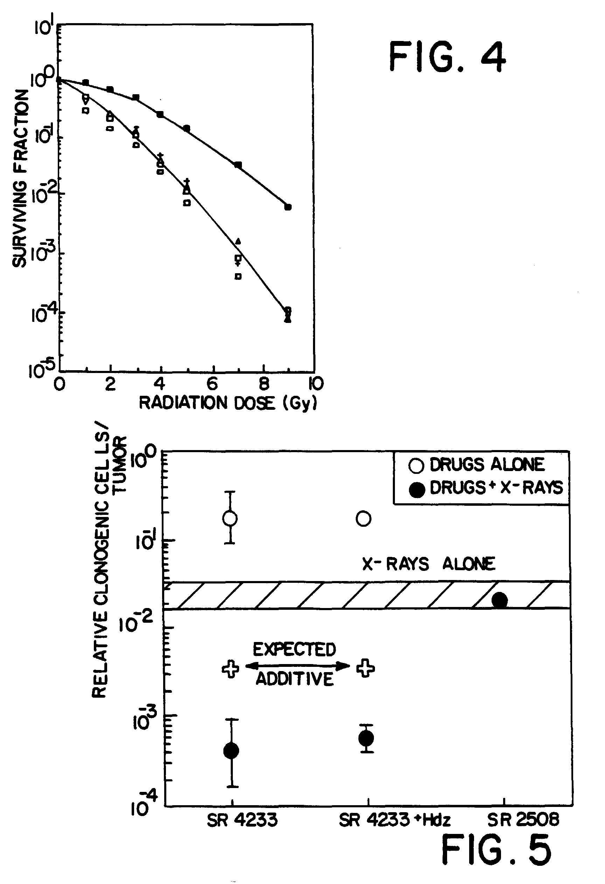

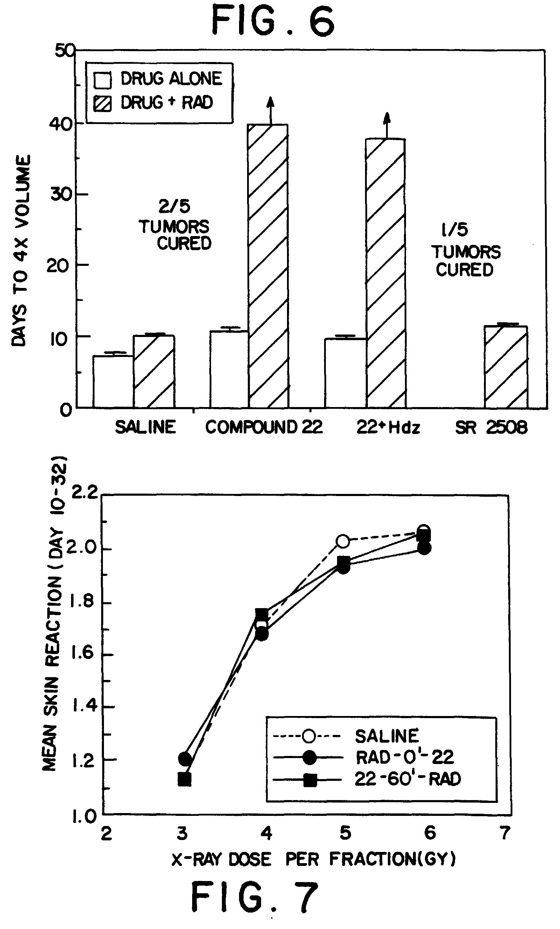

## Claims

1. Use of a compound of the formula 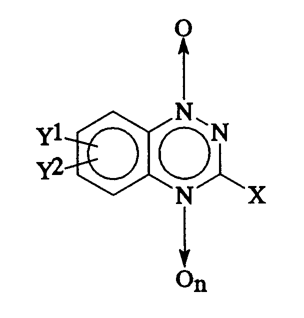 wherein
X is hydrocarbyl (1-4C) substituted with OH, NH₂, alkoxy (1-4C), or halogen; or X is NHR or NRR wherein each R is independently an alkyl of 1-4 carbon atoms substituted with alkyl (1-4C) secondary amino, dialkyl (1-4C) tertiary amino, morpholino, pyrrolidino or piperidino, or an acyl of 1-4 carbon atoms unsubstituted or substituted with OH, NH₂, alkyl (1-4C) secondary amino, dialkyl (1-4C) tertiary amino, morpholino, pyrrolidino, piperidino, alkoxy (1-4C), or halogen, or wherein in the case of NRR the two R groups may be linked together to form a morpholino, pyrrolidino or piperidino ring;
n is 1; and
Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl (1-4C) secondary amino, dialkyl (1-4C) tertiary amino, morpholino, pyrrolidino, piperidino, alkoxy (1-4C) or halogen substituents, or a pharmacologically acceptable salt thereof in the manufacture of a medicament for the treatment of the human or animal body for the selective killing of hypoxic tumor cells.

2. Use according to claim 1 wherein X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring.

3. Use according to claim 2 wherein m is 1 or 2 and Y¹ and Y² are independently selected from the group consisting of H and nitro provided that one but not both of Y¹ and Y² are H.

4. Use of a compound of the formula 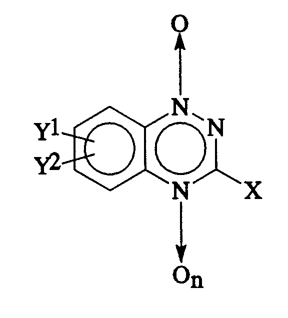 wherein
X is NH₂, NHR or NRR wherein each R is independently an alkyl of 1-4 carbon atoms or acyl of 1-4 carbon atoms, or wherein in the case of NRR the two R groups may be linked together to form a morpholino, pyrrolidino or piperidino ring, and wherein R is unsubstituted or substituted with OH, NH₂, alkyl (1-4C) secondary amino, dialkyl (1-4C) tertiary amino, morpholino, pyrrolidino, piperidino, alkoxy (1-4C), or halogen;
n is 1; and
Y¹ and Y² are chosen such that one but not both may be hydrogen, and one or both may be independently nitro; hydrocarbyl (1-14C) including cyclic and unsaturated hydrocarbyl, substituted with 1 or 2 substituents selected from the group consisting of pyrrolidino, piperidino, acetylaminoalkyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', O(SO)R', or O(POR')R' in which R' is a hydrocarbyl (1-4C) substituted with pyrrolidino or piperidino substituents, or a pharmacologically acceptable salt thereof in the manufacture of a medicament for the treatment of the human or animal body for the selective killing of hypoxic tumor cells.

5. Use according to claim 4, wherein X is NH₂.

6. Use according to claim 5, wherein Y¹ is H and Y² is nitro.

7. Use of a compound of the formula 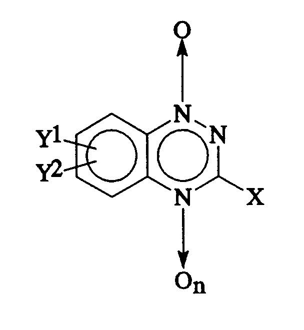 wherein
X is H or hydrocarbyl (1-4C);
n is 1; and
Y¹ and Y² are chosen such that one but not both may be H, and one or both may be independently nitro; hydrocarbyl (1-14C) including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or pyrrolidino, piperidino, NH₂, NHR', NR'R', O(CO)R', NH(CO)R', O(SO)R', or O(POR')R' in which R' is a hydrocarbyl (1-4C) which may be substituted with alkyl(1-4C) secondary amino, dialkyl (1-4C) tertiary amino, pyrrolidino or piperidino substituents with the proviso that when X is H, methyl or ethyl and one of Y¹ and Y² is H, the other of Y¹ and Y² is not NH₂, or a pharmacologically acceptable salt thereof in the manufacture of a medicament for the treatment of the human or animal body for the selective killing of hypoxic cells.

8. Use according to claim 7 wherein X is H.

9. Use according to claim 7 wherein X is hydrocarbyl (1-4C).

10. Use of a compound of the formula: 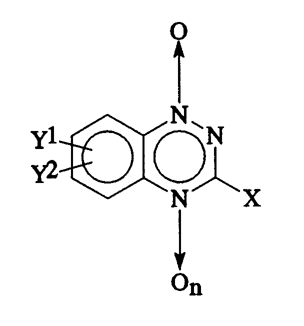 wherein
X is halogen; NHR or NRR, wherein the R groups are independently selected from alkyl (1-4C) substituted with lower alkyl (1-4C) secondary amino or dialkyl (1-4C) tertiary amino groups, acyl (1-4C) and acyl (1-4C) substituted with OH, NH₂, lower alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring; or X is hydrocarbyl (1-4a) substituted with OH, NH₂, NHR or NRR, wherein the R groups are independently selected from substituted alkyl (1-4C) and substituted or unsubstituted acyl (1-4C), and the R groups are substituted with substituents selected from alkyl (1-4C) secondary and dialkyl (1-4C) tertiary amino groups, and in the case of NRR, the two R's can be linked together directly or through a bridge oxygen into a morpholino ring, pyrrolidino ring or piperidino ring;
n is 0 or 1; and
Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents; or a pharmacologically acceptable salt thereof in the manufacture of a medicament for the treatment of the human or animal body for the radiosensitizing of hypoxic tumor cells.

11. Use according to claim 10 wherein X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring.

12. Use according to claim 11 wherein m is 1 or 2 and Y¹ and Y² are independently selected from the group consisting of H and nitro.

13. Use of a compound of the formula: 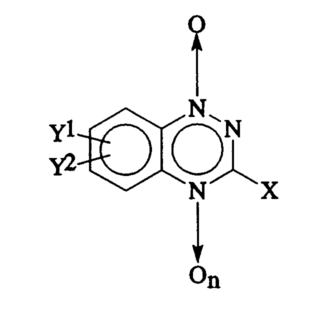 wherein
X is OH; alkoxy (1-4C); NH₂; NHR; NRR; H; hydrocarbyl (1-4C); or hydrocarbyl (1-4C) substituted with OH, NH₂, NHR or NRR; wherein the R groups are independently alkyl (1-4C) or acyl-(1-4C) and are unsubstituted or substituted with OH, NH₂, lower alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring;
n is 0 or 1; and
Y¹ and Y² are chosen such that one but not both may be H, and one or both may be independently nitro; hydrocarbyl (1-14C), including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with pyrrolidino or piperidino substituents with the proviso that when n is 1, X is H, methyl or ethyl and one of Y¹ and Y² is H, the other of Y¹ and Y² is not NH₂; or a pharmacologically acceptable salt thereof in the manufacture of a medicament for the treatment of the human or animal body for the radiosensitizing of hypoxic tumor cells.

14. Use according to claim 13 wherein X is OH or alkoxy (1-4C).

15. Use according to claim 13 wherein X is NH₂, or NHR or NRR.

16. Use according to claim 13 wherein X is NH₂.

17. Use according to claim 16 wherein Y¹ is H, Y² is nitro and n is 1.

18. Use according to claim 14, wherein X is H.

19. Use according to claim 14, wherein X is hydrocarbyl (1-4C).

20. A pharmaceutical composition comprising a compound of the formula 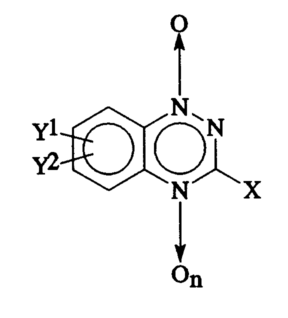 wherein
X is hydrocarbyl (1-4C) substituted with OH, NH₂, alkoxy (1-4C), or halogen; or X is NHR or NRR wherein each R is independently an alkyl of 1-4 carbon atoms substituted with alkyl (1-4C) secondary amino, dialkyl (1-4C) tertiary amino, morpholino, pyrrolidino or piperidino, or an acyl of 1-4 carbon atoms substituted with OH, NH₂, alkyl (1-4C) secondary amino, dialkyl (1-4C) tertiary amino, morpholino, pyrrolidino or piperidino, or wherein in the case of NRR the two R groups may be linked together to form a pyrrolidino ring;
n is 1; and
Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C) or halogen substituents, or a pharmacologically acceptable salt thereof for use in a method of treatment of the human or animal body for the selective killing of hypoxic tumor cells.

21. A composition according to claim 20, wherein X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring.

22. A composition according to claim 21, wherein m is 1 or 2 and Y¹ and Y² are independently selected from the group consisting of H and nitro provided that one but not both of Y¹ and Y² are H.

23. A pharmaceutical composition comprising a compound of the formula 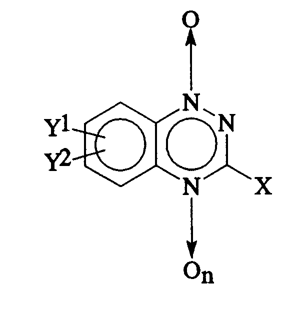 wherein
X is NH₂, NHR or NRR wherein each R is independently an alkyl of 1-4 carbon atoms or acyl of 1-4 carbon atoms, or wherein in the case of NRR the two R groups may be linked together to form a morpholino, pyrrolidino or piperidino ring, and wherein R is unsubstituted or substituted with OH, NH₂, alkyl (1-4C) secondary amino, dialkyl (1-4C) tertiary amino, morpholino, pyrrolidino, piperidino, alkoxy (1-4C), or halogen;
n is 1; and
Y¹ and Y² are chosen such that one but not both may be hydrogen, and one or both may be independently nitro; hydrocarbyl (1-14C) including cyclic and unsaturated hydrocarbyl, substituted with 1 or 2 substituents selected from the group consisting of pyrrolidino, piperidino, acetylaminoalkyl (1-4C), alkylsulfonyl (1-4C), or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', O(SO)R', or O(POR')R' in which R' is a hydrocarbyl (1-4C) substituted with pyrrolidino or piperidino substituents, or a pharmacologically acceptable salt thereof for use in a method of treatment of the human or animal body for the selective killing of hypoxic tumor cells.

24. A composition according to claim 23, wherein X is NH₂.

25. A composition according to claim 24 wherein Y¹ is H and Y² is nitro.

26. A pharmaceutical composition comprising a compound of the formula 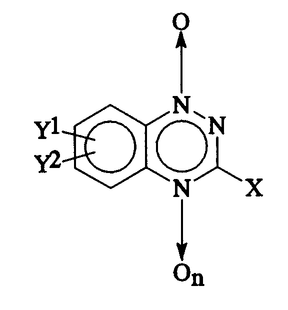 wherein
X is H or hydrocarbyl (1-4C);
n is 1; and
Y¹ and Y² are chosen such that one but not both may be H, and one or both may be independently nitro; hydrocarbyl (1-14C) including cyclic and unsaturated hydrocarbyl, optionally substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or pyrrolidino, piperidino, NH₂, NHR', NR'R', O(CO)R', NH(CO)R', O(SO)R', or O(POR')R' in which R' is a hydrocarbyl (1-4C) which may be substituted with alkyl-(1-4C) secondary amino, dialkyl (1-4C) tertiary amino, pyrrolidino or piperidino substituents; or a pharmacologically acceptable salt thereof, for use in a method of treatment of the human or animal body for the selective killing of hypoxic cells.

27. A composition according to claim 26 wherein X is H.

28. A composition according to claim 26, wherein X is hydrocarbyl (1-4C).

29. A pharmaceutical composition comprising a compound of the formula: 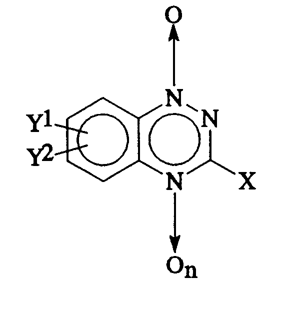 wherein
X is halogen; NHR or NRR, wherein the R groups are independently selected from alkyl (1-4C) substituted with lower alkyl (1-4C) secondary amino or dialkyl (1-4C) tertiary amino groups, and acyl (1-4C) substituted with OH, NH₂, or lower alkyl (1-4C) secondary or dialkyl (1-4C) tertiary amino groups, and in the case of NRR, the two R's can be linked together directly into a pyrrolidino ring; or X is hydrocarbyl (1-4C) substituted with OH, NH₂, NHR or NRR, wherein the R groups are independently selected from substituted alkyl (1-4C) and substituted acyl (1-4C), and the R groups are substituted with substituents selected from alkyl (1-4C) secondary and dialkyl (1-4C) tertiary amino groups, and in the case of NRR, the two R's can be linked together directly into a pyrrolidino ring;
n is 0 or 1; and
Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl-(1-4C) secondary amino, dialkyl (1-4C) tertiary amino, morpholino, pyrrolidino, piperidino, alkoxy (1-4C), or halogen substituents; or a pharmacologically acceptable salt thereof for use in a method of treatment of the human or animal body for the radiosensitizing of hypoxic tumor cells.

30. A composition according to claim 29 wherein X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring.

31. A composition according to claim 30, wherein m is 1 or 2 and Y¹ and Y² are independently selected from the group consisting of H and nitro.

32. A pharmaceutical composition comprising a compound of the formula: 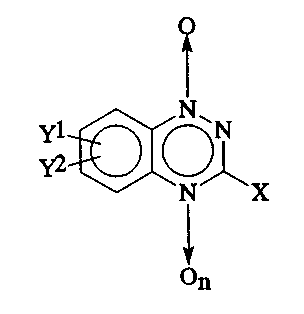 wherein
X is OH; alkoxy (1-4C); NH₂; NHR; NRR; H; hydrocarbyl (1-4C); or hydrocarbyl (1-4C) substituted with OH, NH₂, NHR or NRR; wherein the R groups are independently alkyl (1-4C) or acyl (1-4C) and are unsubstituted or substituted with OH, NH₂, lower alkyl (1-4C) secondary and dialkyl (1-4C) tertiary amino groups, alkoxy (1-4C) or halogen, and in the case of NRR, the two R's can be linked together directly or through a bridge oxygen into a morpholino ring, pyrrolidino ring or piperidino ring;
n is 0 or 1; and
Y¹ and Y² are chosen such that one but not both may be H, and one or both may be independently nitro; hydrocarbyl (1-14C), including cyclic and unsaturated hydrocarbyl, substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with pyrrolidino or piperidino substituents with the proviso that when n is 1, X is H, methyl or ethyl and one of Y¹ and Y² is H, the other of Y¹ and Y² is not NH₂; or a pharmacologically acceptable salt thereof for use in a method of treatment of the human or animal body for the radiosensitizing of hypoxic tumor cells.

33. A composition according to claim 32, wherein X is OH or alkoxy (1-4C).

34. A composition according to claim 32, wherein X is NH₂, or NHR or NRR.

35. A composition according to claim 32, wherein X is NH₂.

36. A composition according to claim 35, wherein Y¹ is H, Y² is nitro, and n is 1.

37. A composition according to claim 32, wherein X is H.

38. A composition according to claim 32, wherein X is hydrocarbyl (1-4C).

39. A compound having the structural formula: 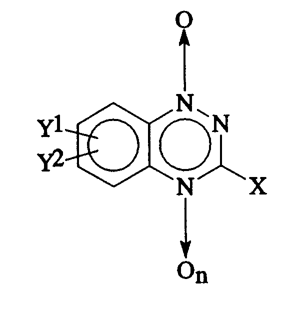 wherein
X is NHR or NRR wherein each R is independently a substituted alkyl of 1-4 carbon atoms, or substituted acyl of 1-4 carbon atoms, or where the two R groups are alkyls linked together to form a pyrrolidino or piperidino ring or linked through an oxygen to form a morpholino ring, and the R groups are substituted with alkyl (1-4C) secondary amino, dialkyl (1-4C) tertiary amino, pyrrolidino or piperidino substituents;
n is 1; and
Y¹ and Y² are independently H; nitro; halogen; hydrocarbyl (1-14C) including cyclic and unsaturated hydrocarbyl, unsubstituted or substituted with 1 or 2 substituents selected from the group consisting of halogen, hydroxy, epoxy, alkoxy (1-4C), alkylthio (1-4C), primary amino (NH₂), lower alkyl (1-4C) secondary amino, dialkyl (1-4C) tertiary amino, morpholino, pyrrolidino, piperidino, acyloxy (1-4C), acylamido (1-4C) and thio analogs thereof acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) which is unsubstituted or substituted with OH, NH₂, alkyl (1-4C) secondary amino, dialkyl (1-4C) tertiary amino, morpholino, pyrrolidino, piperidino, alkoxy (1-4C), or halogen substituents;
or a pharmacologically acceptable salt thereof.

40. A compound according to claim 39, wherein Y¹ and Y² are both H.

41. A compound according to claim 39, wherein X is -NH-CH₂-(CH₂)ₘ-CH₂-NR₁R₂ wherein m is an integer in the range of 0-4 inclusive, and R₁ and R₂ are independently selected from hydrogen or lower alkyls or together form a piperidino or pyrrolidino ring.

42. A compound according to claim 41, wherein m is 1 or 2 and Y¹ and Y² are independently selected from the group consisting of H and nitro.

43. A compound having the structural formula: 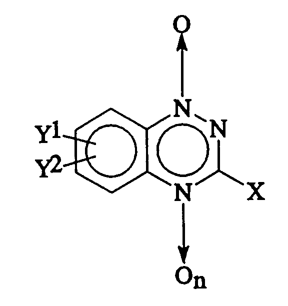 wherein
X is hydrogen or hydrocarbyl (2-4C); or X is OH, alkoxy (1-4C), NHR or NRR where each R is independently an alkyl of 1-4 carbon atoms, or acyl of 1-4 carbon atoms, or where the two R groups are alkyls linked together to form a pyrrolidino or piperidino ring or linked through an oxygen to form a morpholino ring, and the R groups may be further substituted with OH, NH₂, alkyl (1-4C) secondary amino, dialkyl (1-4C) tertiary amino, pyrrolidino, piperidino, alkoxy (1-4C), or halogen substituents;
n is 1; and
Y¹ and Y² are chosen such that one but not both may be hydrogen and one or both independently may be nitro; hydrocarbyl (1-14C), including cyclic and unsaturated hydrocarbyl, said hydrocarbyl being substituted with 1 or 2 substituents selected from the group consisting of pyrrolidino, piperidino, acetylaminoalkyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with one or more pyrrolidino or piperidino substituents with the proviso that when X is H or ethyl and one of Y¹ and Y² is H, the other of Y¹ and Y² is not NH₂;
or a pharmacologically acceptable salt thereof.

44. A compound according to claim 43, wherein X is OH or alkoxy,

45. A compound according to claim 43, wherein X is NRR.

46. A compound according to claim 43, wherein X is H.

47. A compound according to claim 43, wherein X is hydrocarbyl (2-4C).

48. A compound having the structural formula: 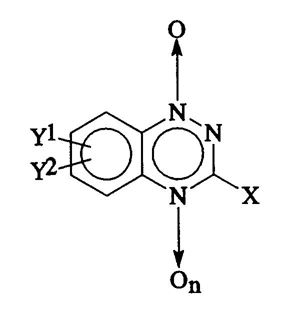 wherein
X is NH₂; or hydrocarbyl (2-4C) substituted with OH, NH₂, alkoxy (1-4C) or halogen substituents;
n is 1; and
Y¹ and Y² are chosen such that one but not both may be hydrogen, and one or both may be independently nitro, saturated or unsaturated hydrocarbyl of 7-14C, or unsaturated hydrocarbyl of 2-6C, said hydrocarbyl groups being substituted with 1 or 2 substituents selected from the group consisting of lower alkyl (1-4C) secondary amino, dialkyl (1-4C) tertiary amino, pyrrolidino, piperidino, acetylaminoalkyl (1-4C), carboxy, alkoxycarbonyl (1-4C), carbamyl, alkylcarbamyl (1-4C), alkylsulfonyl (1-4C) or alkylphosphonyl (1-4C), wherein the hydrocarbyl can optionally be interrupted by a single ether (-O-) linkage; or morpholino, pyrrolidino, piperidino, NH₂, NHR', NR'R', O(CO)R', NH(CO)R', O(SO)R', or O(POR')R' in which R' is a hydrocarbyl (1-4C) substituted with one or more alkyl (1-4C) secondary amino, dialkyl (1-4C) tertiary amino, pyrrolidino or piperidino substituents;
or a pharmacologically acceptable salt thereof.

49. A compound according to claim 48, wherein Y¹ is H and Y² is saturated or unsaturated hydrocarbyl of 7-14C.

50. A compound according to claim 48, wherein Y¹ is H and Y² is unsaturated hydrocarbyl of 2-6C.

51. A compound according to claim 48, wherein Y¹ is H and Y² is nitro.

## Patentansprüche

1. Verwendung einer Verbindung der Formel: 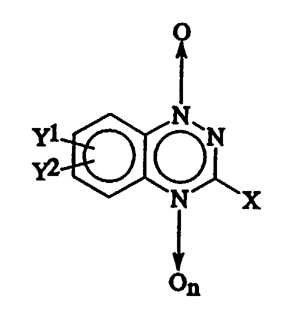 worin
X Hydrocarbyl (1-4C) ist, substituiert mit OH, NH₂, Alkoxy (1-4C) oder Halogen; oder X NHR oder NRR ist, worin jedes R unabhängig voneinander ein Alkyl mit 1-4 Kohlenstoffatomen ist, substituiert mit Alkyl-(1-4C)-sekundärem Amino, Dialkyl-(1-4C)-tertiärem Amino, Morpholino, Pyrrolidino oder Piperidino, oder ein Acyl mit 1-4 Kohlenstoffatomen, unsubstituiert oder substituiert mit OH, NH₂, Alkyl-((1-4C)-sekundärem Amino, Dialkyl-(1-4C)-tertiärem Amino, Morpholino, Pyrrolidino, Piperidino, Alkoxy (1-4C) oder Halogen, oder worin im Falle von NRR die beiden R-Gruppen miteinander verknüpft sein können zur Bildung eines Morpholino-, Pyrrolidino- oder Piperidino-Rings;
n 1 ist; und
Y¹ und Y² unabhängig voneinander H; Nitro; Halogen; Hydrocarbyl (1-14C) sind; einschließlich cyclisches und ungesättigtes Hydrocarbyl, unsubstituiert oder substituiert mit 1 oder 2 Substituenten, gewählt aus der Gruppe, bestehend aus Halogen, Hydroxy, Epoxy, Alkoxy (1-4C), Alkylthio (1-4C), primäres Amino (NH₂), niederes Alkyl-(1-4C)-sekundäres Amino, Dialkyl-(1-4C)-tertiäres Amino, Morpholino, Pyrrolidino, Piperidino, Acyloxy (1-4C), Acylamido (1-4C) und deren Thio-Analoga, Acetylaminoalkyl (1-4C), Carboxy, Alkoxycarbonyl (1-4C), Carbamyl, Alkylcarbamyl (1-4C), Alkylsulfonyl (1-4C) oder Alkylphosphonyl (1-4C), worin das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', O(SO)R' oder O(POR')R', wobei R' ein Hydrocarbyl (1-4C) ist, das unsubstituiert oder substituiert ist mit OH, NH2, Alkyl-(1-4C)-sekundärem Amino, Dialkyl-(1-4C)-tertiärem Amino, Morpholino, Pyrrolidino, Piperidino, Alkoxy (1-4C) oder Halogen-Substituenten, oder ein pharmakologisch geeignetes Salz davon in der Herstellung eines Medikaments zur Behandlung des menschlichen oder tierischen Körpers zur selektiven Abtötung hypoxischer Tumorzellen.

2. Verwendung nach Anspruch 1, worin X -NH-CH₂-(CH₂)ₘ-CH₂-NR₁R₂ ist, worin m eine ganze Zahl im Bereich von 0-4 einschließlich ist, und R₁ und R₂ unabhängig voneinander gewählt sind aus Wasserstoff oder niederen Alkylen oder zusammen einen Piperidino- oder Pyrrolidino-Ring bilden.

3. Verwendung nach Anspruch 2, worin m 1 oder 2 ist und Y₁ und Y₂ unabhängig voneinander gewährt sind aus der Gruppe, bestehend aus H und Nitro, vorausgesetzt, daß eines, jedoch nicht beide von Y₁ und Y₂ H ist.

4. Verwendung einer Verbindung der Formel: 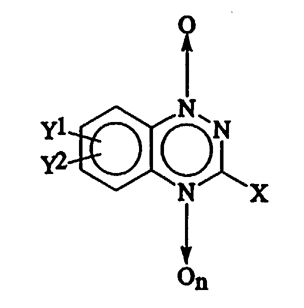 worin
X NH₂, NHR oder NRR ist, worin jedes R unabhängig voneinander ein Alkyl mit 1-4 Kohlenstoffatomen oder Acyl mit 1-4 Kohlenstoffatomen ist, oder worin im Falle von NRR die beiden R-Gruppen miteinander verknüpft sein können zur Bildung eines Morpholino-, Pyrrolidino- oder Piperidino-Rings, und worin R unsubstituiert oder substituiert mit OH, NH₂, Alkyl-(1-4C)-sekundärem Amino, Dialkyl-(1-4C)-tertiärem Amino, Morpholino, Pyrrolidino, Piperidino, Alkoxy (1-4C) oder Halogen ist;
n 1 ist; und
Y¹ und Y² so gewählt sind, daß eines, jedoch nicht beide Wasserstoff sein können, und eines oder beide unabhängig voneinander Nitro; Hydrocarbyl (1-14C) sein kann, einschließlich cyclischem und ungesättigtem Hydrocarbyl, sein können, substituiert mit 1 oder 2 Substituenten, gewährt aus der Gruppe, bestehend aus Pyrrolidino, Piperidino, Acetylaminoalkyl (1-4C), Alkylsulfonyl (1-4C) oder Alkylphosphonyl (1-4C), wobei das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', O(SO)R' oder O(POR')R', wobei R' ein Hydrocarbyl (1-4C) ist, substituiert mit Pyrrolidino- oder Piperidino-Substituenten, oder ein pharmakologisch geeignetes Salz davon in der Herstellung eines Medikaments zur Behandlung des menschlichen oder tierischen Körpers zur selektiven Abtötung hypoxischer Tumorzellen.

5. Verwendung nach Anspruch 4, worin X NH₂ ist.

6. Verwendung nach Anspruch 5, worin Y¹ H und Y² Nitro ist.

7. Verwendung einer Verbindung der Formel: 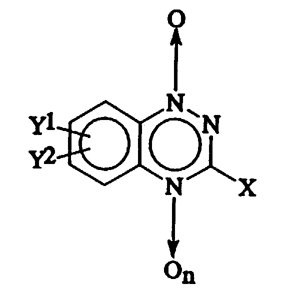 worin
X H oder Hydrocarbyl (1-4C) ist;
n 1 ist; und
Y¹ und Y² derart gewählt sind, daß eines, jedoch nicht beide H sein können, und eines oder beide unabhängig voneinander Nitro; Hydrocarbyl (1-4C) sein können, einschließlich cyclischem und ungesättigtem Hydrocarbyl, substituiert mit 1 oder 2 Substituenten, gewählt aus der Gruppe, bestehend aus Pyrrolidino, Piperidino, Acetylaminoalkyl (1-4C), Alkylsulfonyl (1-4C) oder Alkylphosphonyl (1-4C), wobei das Hydrocarbyl wahlweise durch eine Einzelether-(-O-)-Verknüpfung unterbrochen sein kann; oder Pyrrolidino, Piperidino, NH₂, NHR', NR'R', O(CO)R', NH(CO)R', O(SO)R' oder O(POR')R', wobei R' ein Hydrocarbyl (1-4C) ist, das mit Alkyl-(1-4C)-sekundärem Amino-, Dialkyl-(1-4C)-tertiärem Amino-, Pyrrolidino- oder Piperidino-Substituenten substituiert sein kann, unter der Voraussetzung, daß dann, wenn X H, Methyl oder Ethyl ist und eines von Y¹ und Y² H ist, das andere von Y¹ und Y² nicht NH₂ ist, oder ein pharmakologisch geeignetes Salz davon in der Herstellung eines Medikaments zur Behandlung des menschlichen oder tierischen Körpers zur selektiven Abtötung hypoxischer Tumorzellen.

8. Verwendung nach Anspruch 7, wobei X H ist.

9. Verwendung nach Anspruch 7, wobei X Hydrocarbyl (1-4C) ist.

10. Verwendung einer Verbindung der Formel: 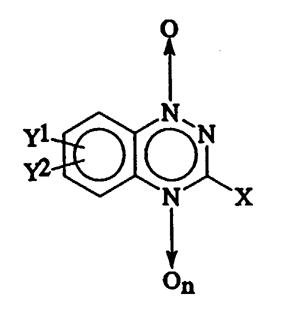 worin
X Halogen; NHR oder NRR ist, worin die R-Gruppen unabhängig voneinander gewährt sind aus Alkyl (1-4C), substituiert mit niederen Alkyl-(1-4C)-sekundären Amine oder Dialkyl-(1-4C)-tertiären Amino-Gruppen, Acyl (1-4C) und Acyl (1-4C), substituiert mit OH, NH₂, niederen Alkyl-(1-4C)-sekundären Amino- oder Dialkyl-(1-4C)-tertiären Amino-Gruppen, Alkoxy (1-4C) oder Halogen, und im Falle von NRR, die beiden R's direkt miteinander oder durch einen Brücken-Sauerstoff in einen Morpholino-Ring, Pyrrolidino-Ring oder Piperidino-Ring verknüpft sein können; oder X Hydrocarbyl (1-4C) ist, substituiert mit OH, NH₂, NHR oder NRR, worin die R-Gruppen unabhängig voneinander gewählt sind aus substituiertem Alkyl (1-4C) und substituiertem oder unsubstituiertem Acyl (1-4C), und die R-Gruppen substituiert sind mit Substituenten, gewährt aus Alkyl-(1-4C)-sekundären und Dialkyl-(1-4C)-tertiären Amino-Gruppen, und im Falle von NRR die beiden R's direkt miteinander oder durch einen Brücken-Sauerstoff in einen Morpholino-Ring, Pyrrolidino-Ring oder Piperidino-Ring verknüpft sein können;
n 0 oder 1 ist; und
Y¹ und Y² unabhängig voneinander H; Nitro; Halogen; Hydrocarbyl (1-14C) sind, einschließlich cyclischem und ungesättigtem Hydrocarbyl, unsubstituiert oder substituiert mit 1 oder 2 Substituenten, gewährt aus der Gruppe, bestehend aus Halogen, Hydroxy, Epoxy, Alkoxy (1-4C), Alkylthio (1-4C), primärem Amino (NH₂), niederem Alkyl-(1-4C)-sekundärem Amino, Dialkyl-(1-4C)tertiärem Amino, Morpholino, Pyrrolidino, Piperidino, Acyloxy (1-4C), Acylamido (1-4C) und deren Thio-Analoga, Acetylaminoalkyl (1-4C), Carboxy, Alkoxycarbonyl (1-4C), Carbamyl, Alkylcarbamyl (1-4C), Alkylsulfonyl (1-4C) oder Alkylphosphonyl (1-4C), worin das Hydrocarbyl wahlweise durch eine Einzelether(-O-)-Verknüpfung unterbrochen sein kann; oder Morpholino, Pyrrolidino, Piperidino, NH₂, NHR', NR'R', O(CO)R', NH(CO)R', O(SO)R' oder O(POR')R', wobei R' ein Hydrocarbyl (1-4C) ist, das unsubstituiert oder substituiert ist mit OH, NH₂, Alkyl-(1-4C)-sekundärem Amino, Dialkyl-(1-4C)-tertiärem Amino, Morpholino, Pyrrolidino, Piperidino. Alkoxy-(1-4C) oder Halogen-Substituenten; oder ein pharmakologisch geeignetes Salz davon in der Herstellung eines Medikaments zur Behandlung des menschlichen oder tierischen Körpers zur Radiosensibilisierung hypoxischer Tumorzellen.

11. Verwendung nach Anspruch 10, wobei X -NH-CH₂-(CH₂)ₘ-CH₂-NR₁R₂ ist, worin m eine ganze Zahl im Bereich von 0-4 einschließlich ist, und R₁ und R₂ unabhängig voneinander gewährt sind aus Wasserstoff oder niederen Alkylen oder zusammen einen Piperidiono- oder Pyrrolidino-Ring bilden.

12. Verwendung nach Anspruch 11, wobei m 1 oder 2 ist und Y¹ und Y² unabhängig voneinander gewährt sind aus der Gruppe, bestehend aus H und Nitro.

13. Verwendung einer Verbindung der Formel: 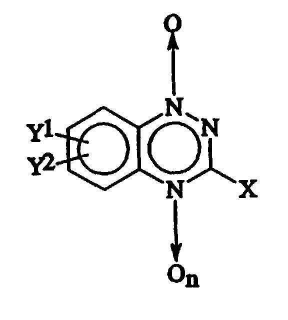 worin
X OH; Alkoxy (1-4C); NH₂; NHR; NRR; H; Hydrocarbyl (1-4C); oder Hydrocarbyl (1-4C) ist, substituiert mit OH, NH₂, NHR oder NRR; wobei die R-Gruppen unabhängig voneinander Alkyl (1-4C) oder Acyl (1-4C) sind und unsubstituiert oder substituiert sind mit OH, NH₂, niederen Alkyl-(1-4C)-sekundären und Dialkyl-(1-4C)-tertiären Amino-Gruppen, Alkoxy (1-4C) oder Halogen, und im Falle von NRR die beiden R's direkt miteinander oder durch einen Brücken-Sauerstoff in einen Morpholino-Ring, Pyrrolidin-Ring oder Piperidino-Ring verknüpft sein können;
n 0 oder 1 ist; und
Y¹ und Y² so gewählt werden, daß eines, jedoch nicht beide H sein können, und eines oder beide unabhängig voneinander Nitro, Hydrocarbyl (1-4C) sein kann, einschließlich cyclisches und ungesättigtes Hydrocarbyl, substituiert mit 1 oder 2 Substituenten, gewählt aus der Gruppe, bestehend aus Pyrrolidino, Piperidio, Acetylaminoalkyl (1-4C), Alkylsulfonyl (1-4C) oder Alkylphosphonyl (1-4C), wobei das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', NH(CO)R', O(SO)R' oder O(POR')R', wobei R' ein Hydrocarbyl (1-4C) ist, substituiert mit Pyrrolidino- oder Piperidino-Substituenten, unter der Voraussetzung, daß dann, wenn n 1 ist, X H, Methyl oder Ethyl ist und eines von Y¹ und Y² H ist, das andere von Y¹ und Y² nicht NH₂ ist; oder ein pharmakologisch geeignetes Salz davon in der Herstellung eines Medikaments zur Behandlung des menschlichen oder tierischen Körpers zur Radiosensibilisierung hypoxischer Tumorzellen.

14. Verwendung nach Anspruch 13, wobei X OH oder Alkoxy (1-4C) ist.

15. Verwendung nach Anspruch 13, wobei X NH₂ oder NHR oder NRR ist.

16. Verwendung nach Anspruch 13, wobei X NH₂ ist.

17. Verwendung nach Anspruch 16, wobei Y¹ H ist, Y² Nitro ist und n 1 ist.

18. Verwendung nach Anspruch 14, wobei X H ist.

19. Verwendung nach Anspruch 14, wobei X Hydrocarbyl (1-4C) ist.

20. Pharmazeutische Zusammensetzung, umfassend eine Verbindung der Formel: 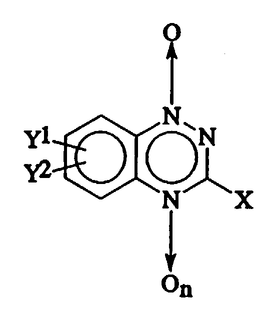 worin
X Hydrocarbyl (1-4C) ist, substituiert mit OH, NH₂, Alkoxy (1-4C) oder Halogen; oder X NHR oder NRR ist, worin jedes R unabhängig voneinander ein Alkyl mit 1-4 Kohlenstoffatomen ist, substituiert mit Alkyl-(1-4C)-sekundärem Amino, Dialkyl-(1-4C)-tertiärem Amino, Morpholino, Pyrrolidino oder Piperidino, oder ein Acyl mit 1-4 Kohlenstoffatomen ist, substituiert mit OH, NH₂, Alkyl-(1-4C)-sekundärem Amino, Dialkyl-1-tertiärem Amino, Morpholino, Pyrrolidino oder Piperidino, oder worin im Falle von NRR die beiden R-Gruppen miteinander verknüpft sein können zur Bildung eines Pyrrolidino-Rings;
n 1 ist; und
Y¹ und Y² unabhängig voneinander H; Nitro; Halogen; Hydrocarbyl (1-14C) sind, einschließlich cyclisches und ungesättigtes Hydrocarbyl, unsubstituiert oder substituiert mit 1 oder 2 Substituenten, gewählt aus der Gruppe, bestehend aus Halogen, Hydroxy, Epoxy, Alkoxy (1-4C), Alkylthio (1-4C), primäres Amino (NH₂), niederes Alkyl-(1-4C)-sekundäres Amino, Dialkyl-(1-4C)-tertiäres Amino, Morpholino, Pyrrolidino, Piperidino, Acyloxy (1-4C), Acylamido (1-4C) und deren Thio-Analoga, Acetylaminoalkyl (1-4C), Carboxy, Alkorycarbonyl (1-4C), Carbamyl, Alkylcarbamyl (1-4C), Alkylsulfonyl (1-4C) oder Alkylphosphonyl (1-4C), wobei das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', NH(CO)R', O(SO)R' oder O(POR')R', wobei R' ein Hydrocarbyl (1-4C) ist, das unsubstituiert oder substituiert ist mit OH, NH₂, Alkyl-(1-4C)-sekundärem Amino, Dialkyl-1-tertiärem Amino, Morpholino, Pyrrolidino, Piperidino, Alkoxy (1-4C) oder Halogen-Substituenten, oder ein pharmakologisch geeignetes Salz davon in der Herstellung eines Medikaments zur Behandlung des menschlichen oder tierischen Körpers zur selektiven Abtötung hypoxischer Tumorzellen.

21. Zusammensetzung nach Anspruch 20, wobei X -NH-CH₂-(CH₂)ₘ-CH₂-NR₁R₂ ist, worin m eine ganze Zahl im Bereich von 0-4 einschließlich ist, und R₁ und R₂ unabhängig voneinander gewählt sind aus Wasserstoff oder niederen Alkylen oder zusammen einen Piperidiono- oder Pyrrolidino-Ring bilden.

22. Zusammensetzung nach Anspruch 21, worin m 1 oder 2 ist und Y¹ und Y² unabhängig voneinander gewählt sind aus der Gruppe, bestehend aus H und Nitro, vorausgesetzt, daß eines, jedoch nicht beide von Y¹ und Y² H ist.

23. Pharmazeutische Zusammensetzung, umfassend eine Verbindung der Formel: 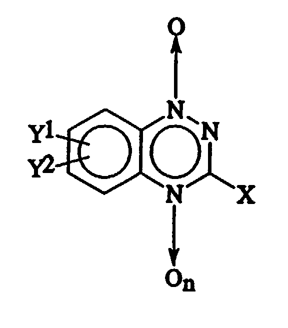 worin
X NH₂, NHR oder NRR ist, wobei jedes R unabhängig voneinander ein Alkyl mit 1-4 Kohlenstoffatomen oder Acyl mit 1-4 Kohlenstoffatomen ist, oder worin im Falle von NRR die beiden R-Gruppen miteinander verknüpft sein können zur Bildung eines Morpholino-, Pyrrolidino- oder Piperidino-Rings, und worin R unsubstituiert oder substituiert mit OH, NH₂, Alkyl-(1-4C)-sekundärem Amino, Dialkyl-(1-4C)-tertiärem Amino, Morpholino, Pyrrolidino, Piperidino, Alkoxy (1-4C) oder Halogen ist;
n 1 ist; und
Y¹ und Y² so gewählt sind, daß eines, jedoch nicht beide Wasserstoff sein können, und eines oder beide unabhängig voneinander Nitro; Hydrocarbyl (1-14C) sein können, einschließlich cyclischem und ungesättigtem Hydrocarbyl, substituiert mit 1 oder 2 Substituenten, gewährt aus der Gruppe, bestehend aus Pyrrolidino, Piperidino, Acetylaminoalkyl (1-4C), Alkylsulfonyl (1-4C) oder Alkylphosphonyl (1-4C), worin das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', O(SO)R' oder O(POR')R', wobei R' ein Hydrocarbyl (1-4C) ist, substituiert mit Pyrrolidino- oder Piperidino-Substituenten, oder ein pharmakologisch geeignetes Salz davon in der Herstellung eines Medikaments zur Behandlung des menschlichen oder tierischen Körpers zur selektiven Abtötung hypoxischer Tumorzellen.

24. Zusammensetzung nach Anspruch 23, worin X NH₂ ist.

25. Verwendung nach Anspruch 24, worin Y¹ H und Y² Nitro ist.

26. Pharmazeutische Zusammensetzung, umfassend eine Verbindung der Formel 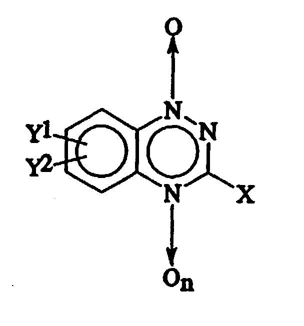 worin
X H oder Hydrocarbyl (1-4C) ist;
n 1 ist; und
Y¹ und Y² derart gewährt sind, daß eines, jedoch nicht beide H sein können, und eines oder beide unabhängig voneinander Nitro; Hydrocarbyl (1-4C) sein können, einschließlich cyclischem und ungesättigtem Hydrocarbyl, wahlweise substituiert mit 1 oder 2 Substituenten, gewährt aus der Gruppe, bestehend aus Pyrrolidino, Piperidino, Acetylaminoalkyl (1-4C), Alkylsulfonyl (1-4C) oder Alkylphosphonyl (1-4C), wobei das Hydrocarbyl wahlweise durch eine Einzelether-(-O-)-Verknüpfung unterbrochen sein kann; oder Pyrrolidino, Piperidino, NH₂, NHR', NR'R', O(CO)R', NH(CO)R', O(SO)R' oder O(POR')R', wobei R' ein Hydrocarbyl (1-4C) ist, das mit Alkyl-(1-4C)-sekundärem Amino, Dialkyl-(1-4C)-tertiärem Amino, Pyrrolidino- oder Piperidino-Substituenten substituiert sein kann; oder ein pharmakologisch geeignetes Salz davon in der Herstellung eines Medikaments zur Behandlung des menschlichen oder tierischen Körpers zur selektiven Abtötung hypoxischer Tumorzellen.

27. Zusammensetzung nach Anspruch 26, wobei X H ist.

28. Zusammensetzung nach Anspruch 26, wobei X Hydrocarbyl (1-4C) ist.

29. Pharmazeutische Zusammensetzung, umfassend eine Verbindung der Formel: 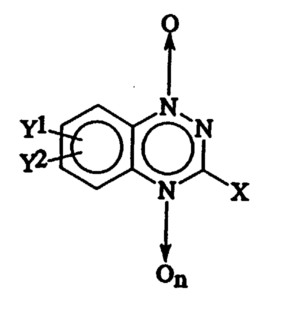 worin
X Halogen; NHR oder NRR ist, wobei die R-Gruppen unabhängig voneinander gewählt sind aus Alkyl (1-4C), substituiert mit niederen Alkyl-(1-4C)-sekundären Amino- oder Dialkyl-(1-4C)tertiären Amino-Gruppen, und Acyl (1-4C), substituiert mit OH, NH₂ oder niederen Alkyl-(1-4C)-sekundären Amino- oder Dialkyl-(1-4C)-tertiären Amino-Gruppen, und im Falle von NRR, die beiden R's direkt miteinander in einen Pyrrolidino-Ring geknüpft sein können; oder X Hydrocarbyl (1-4C) ist, substituiert mit OH, NH₂, NHR oder NRR, worin die R-Gruppen unabhängig voneinander gewählt sind aus substituiertem Alkyl (1-4C) und substituiertem Acyl (1-4C), und die R-Gruppen substituiert sind mit Substituenten, gewährt aus Alkyl-(1-4C)-sekundären und Dialkyl-(1-4C)-tertiären Amino-Gruppen, und im Falle von NRR die beiden R's direkt miteinander in einen Pyrrolidino-Ring geknüpft sein können;
n 0 oder 1 ist; und
Y¹ und Y² unabhängig voneinander H; Nitro; Halogen; Hydrocarbyl (1-14C) sind, einschließlich cyclischem und ungesättigtem Hydrocarbyl, unsubstituiert oder substituiert mit 1 oder 2 Substituenten, gewählt aus der Gruppe, bestehend aus Halogen, Hydroxy, Epoxy, Alkoxy (1-4C), Alkylthio (1-4C), primärem Amino (NH₂), niederem Alkyl-(1-4C)-sekundärem Amino, Dialkyl-(1-4C)-tertiärem Amino, Morpholino, Pyrrolidino, Piperidino, Acyloxy (1-4C), Acylamido (1-4C) und deren Thio-Analoga, Acetylaminoalkyl (1-4C), Carboxy, Alkoxycarbonyl (1-4C), Carbamyl, Alkylcarbamyl (1-4C), Alkylsulfonyl (1-4C) oder Alkylphosphonyl (1-4C), worin das Hydrocarbyl wahlweise durch eine Einzelether(-O-)-Verknüpfung unterbrochen sein kann; oder Morpholino, Pyrrolidino, Piperidino, NH₂, NHR', NR'R', O(CO)R', NH(CO)R', O(SO)R' oder O(POR')R', wobei R' ein Hydrocarbyl (1-4C) ist, das unsubstituiert oder substituiert ist mit OH, NH₂, Alkyl-(1-4C)-sekundärem Amino, Dialkyl-(1-4C)-tertiärem Amino, Morpholino, Pyrrolidino, Piperidino, Alkoxy (1-4C) oder Halogen-Substituenten; oder ein pharmakologisch geeignetes Salz davon in der Herstellung eines Medikaments zur Behandlung des menschlichen oder tierischen Körpers zur Radiosensibilisierung hypoxischer Tumorzellen.

30. Zusammensetzung nach Anspruch 29, wobei X -NH-CH₂-(CH₂)ₘ-CH₂-NR₁R₂ ist, worin m eine ganze Zahl im Bereich von 0-4 einschließlich ist, und R₁ und R₂ unabhängig voneinander gewählt sind aus Wasserstoff oder niederen Alkylen oder zusammen einen Piperidiono- oder Pyrrolidino-Ring bilden.

31. Zusammensetzung nach Anspruch 30, wobei m 1 oder 2 ist und Y¹ und Y² unabhängig voneinander gewählt sind aus der Gruppe, bestehend aus H und Nitro.

32. Pharmazeutische Zusammensetzung, umfassend eine Verbindung der Formel: 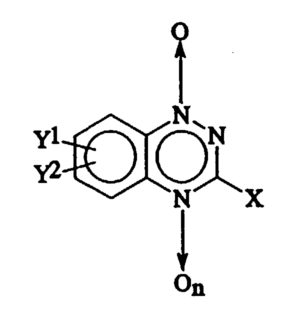 worin
X OH ist; Alkoxy (1-4C); NH₂; NHR; NRR; H; Hydrocarbyl (1-4C); oder Hydrocarbyl (1-4C), substituiert mit OH, NH₂, NHR oder NRR; wobei die R-Gruppen unabhängig voneinander Alkyl (1-4C) oder Acyl (1-4C) sind und unsubstituiert oder substituiert sind mit OH, NH₂, niederen Alkyl-(1-4C)-sekundären und Dialkyl-(1-4C)-tertiären Amino-Gruppen, Alkoxy (1-4C) oder Halogen, und im Falle von NRR die beiden R's direkt miteinander oder durch einen Brücken-Sauerstoff in einen Morpholino-Ring, Pyrrolidino-Ring oder Piperidino-Ring verknüpft sein können;
n 0 oder 1 ist; und
Y¹ und Y² so gewählt werden, daß eines, jedoch nicht beide H sein können, und eines oder beide unabhängig voneinander Nitro; Hydrocarbyl (1-14C) sein können, einschließlich cyclisches und ungesättigtes Hydrocarbyl, substituiert mit 1 oder 2 Substituenten, gewählt aus der Gruppe, bestehend aus Pyrrolidino, Piperidio, Acetylaminoalkyl (1-4C), Alkylsulfonyl (1-4C) oder Alkylphosphonyl (1-4C), wobei das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', NH(CO)R', O(SO)R' oder O(POR')R', wobei R' ein Hydrocarbyl (1-4C) ist, substituiert mit Pyrrolidino- oder Piperidino-Substituenten, unter der Voraussetzung, daß dann, wenn n 1 ist, X H, Methyl oder Ethyl ist und eines von Y¹ und Y² H ist, das andere von Y¹ und Y² nicht NH₂ ist; oder ein pharmakologisch geeignetes Salz davon in der Herstellung eines Medikaments zur Behandlung des menschlichen oder tierischen Körpers zur Radiosensibilisierung hypoxischer Tumorzellen.

33. Zusammensetzung nach Anspruch 32, wobei X OH oder Alkoxy (1-4C) ist.

34. Zusammensetzung nach Anspruch 32, wobei X NH₂ oder NHR oder NRR ist.

35. Zusammensetzung nach Anspruch 32, wobei X NH₂ ist.

36. Zusammensetzung nach Anspruch 35, wobei Y¹ H ist, Y² Nitro ist und n 1 ist.

37. Zusammensetzung nach Anspruch 32, wobei X H ist.

38. Zusammensetzung nach Anspruch 32, wobei X Hydrocarbyl (1-4C) ist.

39. Verbindung mit der Strukturformel: 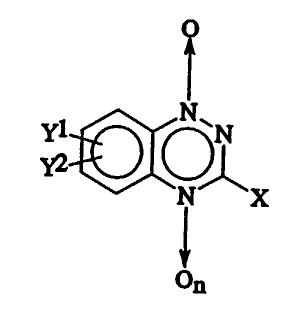 worin
X NHR oder NRR ist, wobei jedes R unabhängig voneinander ein substituiertes Alkyl mit 1-4 Kohlenstoffatomen oder substituiertes Acyl mit 1-4 Kohlenstoffatomen ist, oder worin die beiden R-Gruppen Alkyle sind, die zum Erhalt eines Pyrrolidino- oder Piperidino-Rings miteinander verknüpft sind oder durch einen Sauerstoff zum Erhalt eines Morpholino-Rings verknüpft sind, und die R-Gruppen substituiert sind mit Alkyl-(1-4C)-sekundärem Amino, Dialkyl-(1-4C)-tertiärem Amino, Pyrrolidino- oder Piperidino-Substituenten;
n 1 ist; und
Y¹ und Y² unabhängig voneinander H; Nitro; Halogen; Hydrocarbyl (1-14C) sein können, einschließlich cyclischem oder ungesättigtem Hydrocarbyl, unsubstituiert oder substituiert mit 1 oder 2 Substituenten, gewählt aus der Gruppe, bestehend aus Halogen, Hydroxy, Epoxy, Alkoxy (1-4C), Alkylthio (1-4C), primärem Amino (NH₂), niederem Alkyl-(1-4C)-sekundärem Amino, Dialkyl-(1-4C)-tertiärem Amino, Morpholino, Pyrrolidino, Piperidino, Acyloxy (1-4C), Acylamido (1-4C) und deren Thio-Analoga, Acetylaminoalkyl (1-4C), Carboxy, Alkoxycarbonyl (1-4C), Carbamyl, Alkylcarbamyl (1-4C), Alkylsulfonyl (1-4C) oder Alkylphosphonyl (1-4C), worin das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', NH(CO)R', O(SO)R' oder O(POR')R', wobei R' ein Hydrocarbyl (1-4C) ist, das unsubstituiert oder substituiert ist mit OH, NH₂, Alkyl-(1-4C)-sekundärem Amino, Dialkyl-(1-4C)-tertiärem Amino, Morpholino, Pyrrolidino, Piperidino, Alkoxy (1-4C) oder Halogen-Substituenten;
oder ein pharmakologisch geeignetes Salz davon.

40. Verbindung nach Anspruch 39, wobei Y¹ und Y² beide H sind.

41. Verbindung nach Anspruch 39, worin X -NH-CH₂-(CH₂)ₘ-CH₂NR₁R₂ ist, worin m eine ganze Zahl im Bereich von 0-4 einschließlich ist, und R₁ und R₂ unabhängig voneinander gewählt werden aus Wasserstoff oder niederen Alkylen oder zusammen einen Piperidiono- oder Pyrrolidino-Ring bilden.

42. Verbindung nach Anspruch 41, worin m 1 oder 2 ist und Y¹ und Y² unabhängig voneinander gewählt werden aus der Gruppe, bestehend aus H und Nitro.

43. Verbindung der Strukturformel: 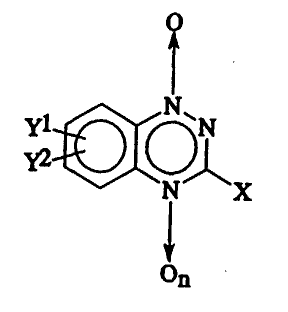 worin
X Wasserstoff oder Hydrocarbyl (2-4C) ist; oder X OH, Alkoxy (1-4C), NHR oder NRR ist, wobei jedes R unabhängig voneinander ein Alkyl mit 1-4 Kohlenstoffatomen oder Acyl mit 1-4 Kohlenstoffatomen ist, oder worin die beiden R-Gruppen Alkyle sind, die zum Erhart eines Pyrrolidino- oder Piperidino-Rings miteinander verknüpft sind oder durch einen Sauerstoff zum Erhalt eines Morpholino-Rings verknüpft sind, und die R-Gruppen außerdem substituiert sein können mit OH, NH₂, Alkyl-(1-4C)-sekundärem Amino, Dialkyl-(1-4C)-tertiärem Amino, Pyrrolidino, Piperidino, Alkoxy (1-4C) oder Halogen-Substituenten;
n 1 ist; und
Y¹ und Y² so gewählt sind, daß eines, jedoch nicht beide Wasserstoff sein können, und eines oder beide unabhängig voneinander Nitro; Hydrocarbyl (1-4C) sein können, einschließlich cyclischem und ungesättigtem Hydrocarbyl, wobei das Hydrocarbyl substituiert ist mit 1 oder 2 Substituenten, gewählt aus der Gruppe, bestehend aus Pyrrolidino, Piperidino, Acetylaminoalkyl (1-4C), Alkylsulfonyl (1-4C) oder Alkylphosphonyl (1-4C), worin das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', O(SO)R' oder O(POR')R', wobei R' ein Hydrocarbyl (1-4C) ist, substituiert mit ein oder mehreren Pryrrolidino- oder Piperidino-Substituenten, unter der Voraussetzung, daß dann, wenn X H oder Ethyl ist und eines von Y¹ und Y² H ist, das andere von Y¹ und Y² nicht NH₂ ist;
oder ein pharmakologisch geeignetes Salz davon.

44. Verbindung nach Anspruch 43, wobei X OH oder Alkoxy ist.

45. Verbindung nach Anspruch 43, wobei X NRR ist.

46. Verbindung nach Anspruch 43, wobei X H ist.

47. Verbindung nach Anspruch 43, wobei X Hydrocarbyl (2-4C) ist.

48. Verbindung mit der Strukturformel: 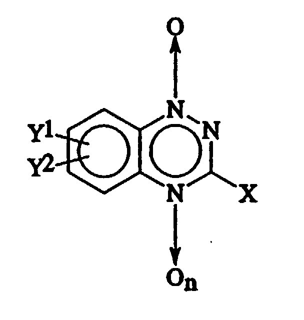 worin
X NH₂ ist; oder Hydrocarbyl (2-4C), substituiert mit OH, NH₂, Alkoxy (1-4C) oder Halogen-Substituenten;
n 1 ist; und
Y¹ und Y² derart gewählt sind, daß eines, jedoch nicht beide Wasserstoff sein können, und eines oder beide unabhängig voneinander Nitro, gesättigtes oder ungesättigtes Hydrocarbyl mit 7-14C oder ungesättigtes Hydrocarbyl mit 2-6C sein können, wobei die Hydrocarbyl-Gruppen substituiert sind mit 1 oder 2 Substituenten, gewählt aus der Gruppe, bestehend aus niederem Alkyl-(1-4C)-sekundärem Amino, Dialkyl-(1-4C)-tertiärem Amino, Pyrrolidino, Piperidino, Acetylaminoalkyl (1-4C), Carboxy, Alkoxycarbonyl (1-4C), Carbamyl, Alkylcarbamyl (1-4C), Alkylsulfonyl (1-4C) oder Alkylphosphonyl (1-4C), wobei das Hydrocarbyl wahlweise durch eine Einzelether-(-O-)-Verknüpfung unterbrochen sein kann; oder Morpholino, Pyrrolidino, Piperidino, NH₂, NHR', NR'R', O(CO)R', NH(CO)R', O(SO)R' oder O(POR')R'. wobei R' ein Hydrocarbyl (1-4C) ist, das mit ein oder mehreren Alkyl-(1-4C)-sekundären Amino-, Dialkyl-(1-4C)-tertiären Amino-, Pyrrolidino- oder Piperidino-Substituenten substituiert sein kann;
oder ein pharmakologisch geeignetes Salz davon.

49. Verbindung nach Anspruch 48, wobei Y¹ H ist und Y² gesättigtes oder ungesättigtes Hydrocarbyl mit 7-14C.

50. Verbindung nach Anspruch 48, wobei Y¹ H ist und Y² ungesättigtes Hydrocarbyl mit 2-6C.

51. Verbindung nach Anspruch 48, wobei Y' H und Y² Nitro ist.

## Revendications

1. Utilisation d'un composé de formule 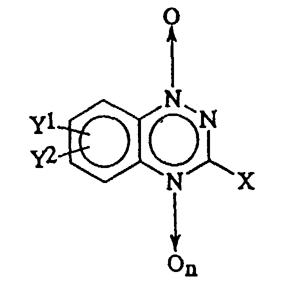 dans laquelle
X est un groupe hydrocarbyle en C₁ à C₄ substitué par OH, NH₂, alcoxy en C₁ à C₄ ou halogène ; ou X est NHR ou NRR, ou chaque R est indépendamment un alkyle de 1 à 4 atomes de carbone substitué par un groupe alkyl (C₁ à C₄)-amino secondaire, dialkyl (C₁ à C₄)-amino tertiaire, morpholino, pyrrolidino ou pipéridino, ou un acyle de 1 à 4 atomes de carbone non substitué ou substitué par OH, NH₂, un groupe alkyl (C₁ à C₄)-amino secondaire, un groupe dialkyl (C₁ à C₄)-amino tertiaire, morpholino, pyrrolidino, pipéridino, alcoxy en C₁ à C₄ ou halogène, ou bien, dans le cas de NRR, les deux groupes R peuvent être liés ensemble pour former un cycle morpholino, pyrrolidino ou pipéridino ;
n est 1 ; et
Y¹ et Y² sont indépendamment H ; nitro ; halogène ; hydrocarbyle en C₁ à C₁₄, y compris hydrocarbyle cyclique et hydrocarbyle insaturé, non substitué ou substitué par 1 ou 2 substituants choisis dans le groupe constitué par halogène, hydroxy, époxy, alcoxy en C₁ à C₄, alkylthio en C₁ à C₄, amino primaire (NH₂), alkyl inférieur (C₁ à C₄)-amino secondaire, dialkyl (C₁ à C₄)-amino tertiaire, morpholino, pyrrolidino, pipéridino, acyloxy en C₁ à C₄, acylamido en C₁ à C₄ et les analogues thio de ceux-ci, acétylaminoalkyle en C₁ à C₄, carboxy, alcoxycarbonyle en C₁ à C₄, carbamyle, alkylcarbamyle en C₁ à C₄, alkyl-sulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ qui est non substitué ou substitué par des substituants OH, NH₂, un groupe alkyl (C₁ à C₄)-amino secondaire, dialkyl (C₁ à C₄)-amino tertiaire, morpholino, pyrrolidino, pipéridino, alcoxy en C₁ à C₄ ou halogène, ou d'un sel pharmacologiquement acceptable de ce composé, dans la fabrication d'un médicament pour le traitement du corps humain ou animal pour tuer sélectivement des cellules tumorales hypoxiques.

2. Utilisation selon la revendication 1, dans laquelle X est -NH-CH₂-(CH₂)ₘ-CH₂-NR₁R₂ où m est un entier dans la gamme de 0 à 4 inclusivement, et R₁ et R₂ sont indépendamment choisis parmi l'hydrogène ou les groupes alkyles inférieurs ou forment ensemble un cycle pipéridino ou pyrrolidino.

3. Utilisation selon la revendication 2, dans laquelle m est 1 ou 2 et Y¹ et Y² sont indépendamment choisis dans le groupe constitué par H et nitro, étant entendu que l'un parmi Y¹ et Y², mais pas les deux, est H.

4. Utilisation d'un composé de formule 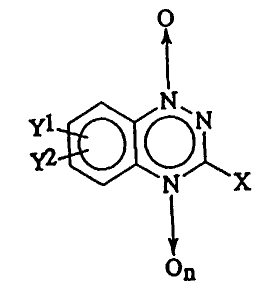 dans laquelle
X est NH₂, NHR ou NRR, où chaque R est indépendamment un alkyle de 1 à 4 atomes de carbone ou un acyle de 1 à 4 atomes de carbone, ou bien, dans le cas de NRR, les deux groupes R peuvent être liés ensemble pour former un cycle morpholino, pyrrolidino ou pipéridino, et où R est non substitué ou substitué par OH, NH₂, un groupe alkyl (C₁ à C₄)-amino secondaire, dialkyl (C₁ à C₄)-amino tertiaire, morpholino, pyrrolidino, pipéridino, alcoxy en C₁ à C₄ ou halogène ;
n est 1 ; et
Y¹ et Y² sont choisis de sorte que l'un, mais pas les deux, peut être de l'hydrogène, et l'un ou les deux peuvent être indépendamment nitro ; hydrocarbyle en C₁ à C₁₄, y compris hydrocarbyle cyclique et hydrocarbyle insaturé, substitué par 1 ou 2 substituants choisis dans le groupe constitué par pyrrolidino, pipéridino, acétylaminoalkyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', O(SO)R' ou O(POR')R' où R' est un groupe hydrocarbyle en C₁ à C₄ substitué par des substituants pyrrolidino ou pipéridino, ou d'un sel pharmacologiquement acceptable de ce composé, dans la fabrication d'un médicament pour le traitement du corps humain ou animal pour tuer sélectivement des cellules tumorales hypoxiques.

5. Utilisation selon la revendication 4, dans laquelle X est NH₂.

6. Utilisation selon la revendication 5, dans laquelle Y¹ est H et Y² est un groupe nitro.

7. Utilisation d'un composé de formule 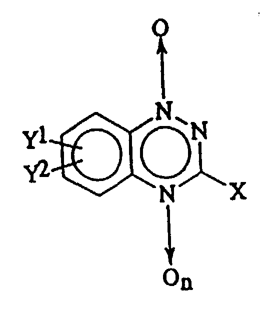 dans laquelle
X est H ou un groupe hydrocarbyle en C₁ à C₄ ;
n est 1 ; et
Y¹ et Y² sont choisis de sorte que l'un, mois pas les deux, peut être H, et l'un ou les deux peuvent être indépendamment nitro ; hydrocarbyle en C₁ à C₁₄, Y compris hydrocarbyle cyclique et hydrocorbyle insaturé, substitué par 1 ou 2 substituants choisis dans le groupe constitué par pyrrolidino, pipéridino, acétylaminoalkyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou pyrrolidino, pipéridino, NH₂, NHR', NR'R', O(CO)R', NH(CO)R', O(SO)R' ou O(POR')R' où R' est un groupe hydrocorbyle en C₁ à C₄ qui peut être substitué par des substituants alkyl (C₁ à C₄)-amino secondaire, dialkyl (C₁ à C₄)-amino tertiaire, pyrrolidino ou pipéridino, étant entendu que lorsque X est H, méthyle ou éthyle et que l'un parmi Y¹ et Y² est H, l'autre parmi Y¹ et Y² n'est pas NH₂, ou d'un sel pharmacologiquement acceptable de ce composé, dans la fabrication d'un médicament pour le traitement du corps humain ou animal pour tuer sélectivement des cellules hypoxiques.

8. Utilisation selon la revendication 7, dans laquelle X est H.

9. Utilisation selon la revendication 7, dans laquelle X est un groupe hydrocarbyle en C₁ à C₄.

10. Utilisation d'un composé de formule 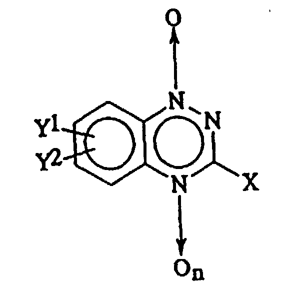 dans laquelle
X est halogène ; NHR ou NRR, où les groupes R sont indépendamment choisis parmi un alkyle en C₁ à C₄ substitué par des groupes alkyl inférieur (C₁ à C₄)-amino secondaire ou dialkyl inférieur (C₁ à C₄)-amino tertiaire, un acyle en C₁ à C₄ et acyle en C₁ à C₄ substitué par ON, NH₂, des groupes alkyl inférieur (C₁ à C₄)-amino secondaire et dialkyl inférieur (C₁ à C₄)-amino tertiaire, alcoxy en C₁ à C₄ ou halogène et, dans le cas de NRR, les deux R peuvent être liés ensemble, directement ou par un pont oxygène, en un cycle morpholino, un cycle pyrrolidino ou un cycle pipéridino ; ou bien X est un groupe hydrocarbyle en C₁ à C₄ substitué par OH, NH₂, NHR ou NRR, où les groupes R sont indépendamment choisis parmi un alkyle en C₁ à C₄ substitué et un acyle en C₁ à C₄ substitué ou non substitué, et les groupes R sont substitués par des substituants choisis parmi les groupes alkyl (C₁ à C₄)-amino secondaire et dialkyl (C₁ à C₄)-amino tertiaire et, dans le cas de NRR, les deux R peuvent être liés ensemble, directement ou par un pont oxygène, en un cycle morpholino, un cycle pyrrolidino ou un cycle pipéridino ;
n est 0 ou 1 ; et
Y¹ et Y² sont indépendamment H ; nitro ; halogène ; hydrocarbyle en C₁ à C₁₄, y compris hydrocarbyle cyclique et hydrocarbyle insaturé, non substitué ou substitué par 1 ou 2 substituants choisis dans le groupe constitué par halogène, hydroxy, époxy, alcoxy en C₁ à C₄, alkylthio en C₁ à C₄, amino primaire (NH₂), alkyl inférieur (C₁ à C₄)-amino secondaire, dialkyl (C₁ à C₄)-amino tertiaire, morpholino, pyrrolidino, pipéridino, acyloxy en C₁ à C₄, acylamido en C₁ à C₄ et les analogues thio de ceux-ci, acétylaminoalkyle en C₁ à C₄, carboxy, alcoxycarbonyle en C₁ à C₄, carbamyle, alkYlcarbamyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ qui est non substitué ou substitué par des substituants OH, NH₂, un groupe alkyl (C₁ à C₄)-amino secondaire, dialkyl (C₁ à C₄)-amino tertiaire, morpholino, pyrrolidino, pipéridino, alcoxy en C₁ à C₄ ou halogène ; ou d'un sel pharmacologiquement acceptable de ce composé, dans la fabrication d'un médicament pour le traitement du corps humain ou animal pour radiosensibiliser des cellules tumorales hypoxiques.

11. Utilisation selon la revendication 10, dans laquelle X est -NH-CH₂-(CH₂)ₘ-CH₂-NR₁R₂ où m est un entier dans la gamme de 0 à 4 inclusivement, et R₁ et R₂ sont indépendamment choisis parmi l'hydrogène et les groupes alkyles inférieurs ou forment ensemble un cycle pipéridino ou pyrrolidino.

12. Utilisation selon la revendication 11, dans laquelle m est 1 ou 2 et Y¹ et Y² sont indépendamment choisis dans le groupe constitué par H et le groupe nitro.

13. Utilisation d'un composé de formule : 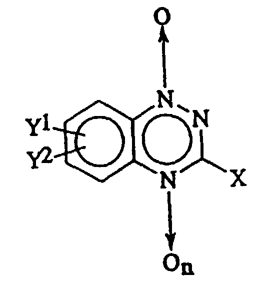 dans laquelle
X est OH ; alcoxy en C₁ à C₄ ; NH₂ ; NHR ; NRR ; H ; hydrocarbyle en C₁ à C₄ ; ou hydrocarbyle en C₁ à C₄ substitué par OH, NH₂, NHR ou NNR ; où les groupes R sont indépendamment un alkyle en C₁ à C₄ ou un acyle en C₁ à C₄ et sont non substitués ou substitués par OH, NH₂, des groupes alkyl inférieur (C₁ à C₄)-amino secondaire et dialkyl inférieur (C₁ à C₄)-amino tertiaire, alcoxy en C₁ à C₄ ou halogène, et, dans le cas de NRR, les deux R peuvent être liés ensemble, directement ou par un pont oxygène, en un cycle morpholino, un cycle pyrrolidino ou un cycle pipéridino ;
n est 0 ou 1 ; et
Y¹ et Y² sont choisis de sorte que l'un, mais pas les deux, peut être H, et l'un ou les deux peuvent être indépendamment nitro ; hydrocarbyle en C₁ à C₁₄, y compris hydrocarbyle cyclique et hydrocarbyle insaturé, substitué par 1 ou 2 substituants choisis dans le groupe constitué par pyrrolidino, pipéridino, acétylaminoalkyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ substitué par des substituants pyrrolidino ou pipéridino, étant entendu que lorsque n est 1, X est H, méthyle ou éthyle, et l'un parmi Y¹ et Y² est H, l'autre parmi Y¹ et Y² n'est pas NH₂ ; ou d'un sel pharmacologiquement acceptable de ce composé, dans la fabrication d'un médicament pour le traitement du corps humain ou animal pour radiosensibiliser des cellules tumorales hypoxiques.

14. Utilisation selon la revendication 13, dans laquelle X est OH ou un groupe alcoxy en C₁ à C₄.

15. Utilisation selon la revendication 13, dans laquelle X est NH₂, ou NHR ou NRR.

16. Utilisation selon la revendication 13, dans laquelle X est NH₂.

17. Utilisation selon la revendication 16, dans laquelle Y¹ est H, Y² est un groupe nitro et n est 1.

18. Utilisation selon la revendication 14, dans laquelle X est H.

19. Utilisation selon la revendication 14, dans laquelle X est un groupe hydrocarbyle en C₁ à C₄.

20. Composition pharmaceutique comprenant un composé de formule 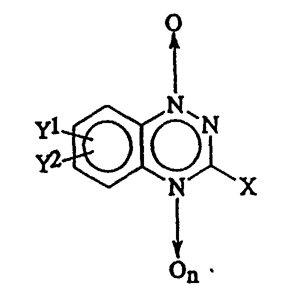 dans laquelle
X est un groupe hydrocarbyle en C₁ à C₄ substitué par OH, NH₂, alcoxy en C₁ à C₄ ou halogène ; ou X est NHR ou NRR, où chaque R est indépendamment un alkyle de 1 à 4 atomes de carbone substitué par un groupe alkyl (C₁ à C₄)-amino secondaire, dialkyl (C₁ à C₄)-amino tertiaire, morpholino, pyrrolidino ou pipéridino, ou un acyle de 1 à 4 atomes de carbone substitué par OH, NH₂, un groupe alkyl (C₁ à C₄)-amino secondaire, dialkyl (C₁ à C₄)-amino tertiaire, morpholino, pyrrolidino ou pipéridino, ou bien, dans le cas de NRR, les deux groupes R peuvent être liés ensemble pour former un cycle pyrrolidino ;
n est 1 ; et
Y¹ et Y² sont indépendamment H ; nitro ; halogène ; hydrocarbyle en C₁ à C₁₄, y compris hydrocarbyle cyclique et hydrocarbyle insaturé, non substitué ou substitué par 1 ou 2 substituants choisis dans le groupe constitué par halogène, hydroxy, époxy, alcoxy en C₁ à C₄, alkylthio en C₁ à C₄, amino primaire (NH₂), alkyl inférieur (C₁ à C₄)-amino secondaire, dialkyl (C₁ à C₄)-amino tertiaire, morpholino, pyrrolidino, pipéridino, acyloxy en C₁ à C₄, acylamido en C₁ à C₄ et les analogues thio de ceux-ci, acétylaminoalkyle en C₁ à C₄, carboxy, alcoxycarbonyle en C₁ à C₄, carbamyle, alkylcarbamyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ qui est non substitué ou substitué par des substituants OH, NH₂, un groupe alkyl (C₁ à C₄)-amino secondaire, dialkyl (C₁ à C₄)-amino tertiaire, morpholino, pyrrolidino, pipéridino, alcoxy en C₁ à C₄ ou halogène, ou un sel pharmacologiquement acceptable de ce composé, destinée à l'utilisation dans une méthode de traitement du corps humain ou animal pour tuer sélectivement des cellules tumorales hypoxiques.

21. Composition selon la revendication 20, dans laquelle X est -NH-CH₂-(CH₂)ₘ-CH₂-NR₁R₂ où m est un entier dans la gamme de 0 à 4 inclusivement, et R₁ et R₂ sont indépendamment choisis parmi l'hydrogène et les groupes alkyles inférieurs ou forment ensemble un cycle pipéridino ou pyrrolidino.

22. Composition selon la revendication 21, dans laquelle m est 1 ou 2 et Y¹ et Y² sont indépendamment choisis dans le groupe constitué par H et le groupe nitro, étant entendu que l'un parmi Y¹ et Y², mais pas les deux, est H.

23. Composition pharmaceutique comprenant un composé de formule 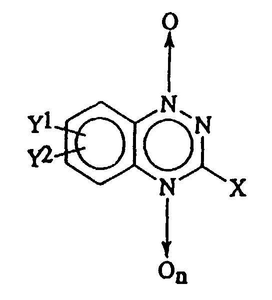 dans laquelle
X est NH₂, NHR ou NRR, où chaque R est indépendamment un alkyle de 1 à 4 atomes de carbone ou un acyle de 1 à 4 atomes de carbone, ou bien, dans le cas de NRR, les deux groupes R peuvent être liés ensemble pour former un cycle morpholino, pyrrolidino ou pipéridino, et où R est non substitué ou substitué par OH, NH₂, un groupe alkyl (C₁ à C₄)-amino secondaire, dialkyl (C₁ à C₄)-amino tertiaire, morpholino, pyrrolidino, pipéridino, alcoxy en C₁ à C₄ ou halogène ;
n est 1 ; et
Y¹ et Y² sont choisis de sorte que l'un, mais pas les deux, peut être de l'hydrogène, et l'un ou les deux peuvent être indépendamment nitro ; hydrocarbyle en C₁ à C₁₄, y compris hydrocarbyle cyclique et hydrocarbyle insaturé, substitué par 1 ou 2 substituants choisis dans le groupe constitué par pyrrolidino, pipéridino, acétylaminoalkyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', O(SO)R' ou O(POR')R' où R' est un groupe hydrocarbyle en C₁ à C₄ substitué par des substituants pyrrolidino ou pipéridino, ou un sel pharmacologiquement acceptable de ce composé, destinée à l'utilisation dans une méthode de traitement du corps humain ou animal pour tuer sélectivement des cellules tumorales hypoxiques.

24. Composition selon la revendication 23, dans laquelle X est NH₂.

25. Composition selon 1a revendication 24, dans laquelle Y¹ est H et Y² est un groupe nitro.

26. Composition pharmaceutique comprenant un composé de formule 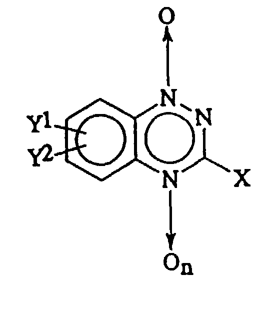 dans laquelle
X est H ou un groupe hydrocarbyle en C₁ à C₄ ;
n est 1 ; et
Y¹ et Y² sont choisis de sorte que l'un, mais pas les deux, peut être H, et l'un ou les deux peuvent être indépendamment nitro ; hydrocarbyle en C₁ à C₁₄, y compris hydrocarbyle cyclique et hydrocarbyle insaturé, facultativement substitué par 1 ou 2 substituants choisis dans le groupe constitué par pyrrolidino, pipéridino, acétylaminoalkyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou pyrrolidino, pipéridino, NH₂, NHR', NR'R', O(CO)R', NH(CO)R', O(SO)R' ou O(POR')R' où R' est un groupe hydrocarbyle en C₁ à C₄ qui peut être substitué par des substituants alkyl (C₁ à C₄)-amino secondaire, dialkyl (C₁ à C₄)-amino tertiaire, pyrrolidino ou pipéridino ; ou un sel pharmacologiquement acceptable de ce composé, destinée à l'utilisation dans une méthode de traitement du corps humain ou animal pour tuer sélectivement des cellules hypoxiques.

27. Composition selon la revendication 26, dans laquelle X est H.

28. Composition selon la revendication 26, dans laquelle X est un groupe hydrocarbyle en C₁ à C₄.

29. Composition pharmaceutique comprenant un composé de formule : 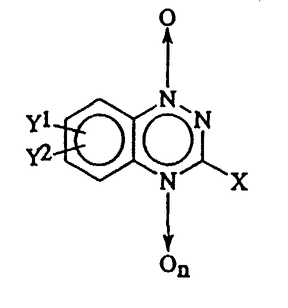 dans laquelle
X est halogène ; NHR ou NRR, où les groupes R sont indépendamment choisis parmi un alkyle en C₁ à C₄ substitué par des groupes alkyl inférieur (C₁ à C₄)-amino secondaire ou dialkyl inférieur (C₁ à C₄)-amino tertiaire, et un acyle en C₁ à C₄ substitué par OH, NH₂, ou des groupes alkyl inférieur (C₁ à C₄)-amino secondaire ou dialkyl inférieur (C₁ à C₄)-amino tertiaire et, dans le cas de NRR, les deux R peuvent être liés ensemble directement en un cycle pyrrolidino ; ou bien X est un groupe hydrocarbyle en C₁ à C₄ substitué par OH, NH₂, NHR ou NRR, où les groupes R sont indépendamment choisis parmi un alkyle en C₁ à C₄ substitué et un acyle en C₁ à C₄ substitué, et les groupes R sont substitués par des substituants choisis parmi les groupes alkyl (C₁ à C₄)-amino secondaire et dialkyl (C₁ à C₄)-amino tertiaire et, dans le cas de NRR, les deux R peuvent être liés ensemble directement en un cycle pyrrolidino ;
n est 0 ou 1 ; et
Y¹ et Y² sont indépendamment H ; nitro ; halogène ; hydrocarbyle en C₁ à C₁₄, y compris hydrocarbyle cyclique et hydrocarbyle insaturé, non substitué ou substitué par 1 ou 2 substituants choisis dans le groupe constitué par halogène, hydroxy, époxy, alcoxy en C₁ à C₄, alkylthio en C₁ à C₄, amino primaire (NH₂), alkyl inférieur (C₁ à C₄)-amino secondaire, dialkyl (C₁ à C₄)-amino tertiaire, morpholino, pyrrolidino, pipéridino, acyloxy en C₁ à C₄, acylamido en C₁ à C₄ et les analogues thio de ceux-ci, acétylaminoalkyle en C₁ à C₄, carboxy, alcoxycarbonyle en C₁ à C₄, carbamyle, alkylcarbamyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ qui est non substitué ou substitué par des substituants OH, NH₂, un groupe alkyl (C₁ à C₄)-amino secondaire, dialkyl (C₁ à C₄)-amino tertiaire, morpholino, pyrrolidino, pipéridino, alcoxy en C₁ à C₄ ou halogène ; ou un sel pharmacologiquement acceptable de ce composé, destinée à l'utilisation dans une méthode de traitement du corps humain ou animal pour radiosensibiliser des cellules tumorales hypoxiques.

30. Composition selon la revendication 29, dans laquelle X est -NH-CH₂-(CH₂)ₘ-CH₂-NR₁R₂ où m est un entier dans la gamme de 0 à 4 inclusivement, et R₁ et R₂ sont indépendamment choisis parmi l'hydrogène ou les groupes alkyles inférieurs ou forment ensemble un cycle pipéridino ou pyrrolidino.

31. Composition selon la revendication 30, dans laquelle m est 1 ou 2 et Y¹ et Y² sont indépendamment choisis dans le groupe constitué par H et le groupe nitro.

32. Composition pharmaceutique comprenant un composé de formule : 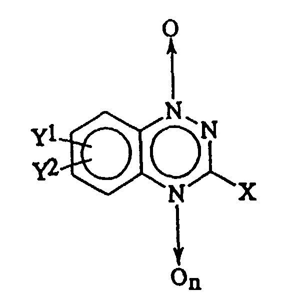 dans laquelle
X est OH ; alcoxy en C₁ à C₄ ; NH₂ ; NHR ; NRR ; H ; hydrocarbyle en C₁ à C₄ ; ou hydrocarbyle en C₁ à C₄ substitué par OH, NH₂, NHR ou NNR ; où les groupes R sont indépendamment un alkyle en C₁ à C₄ ou un acyle en C₁ à C₄ et sont non substitués ou substitués par OH, NH₂, des groupes alkyl inférieur (C₁ à C₄)-amino secondaire et dialkyl (C₁ à C₄)-amino tertiaire, alcoxy en C₁ à C₄ ou halogène, et, dans le cas de NRR, les deux R peuvent être liés ensemble, directement ou par un pont oxygène, en un cycle morpholino, un cycle pyrrolidino ou un cycle pipéridino ;
n est 0 ou 1 ; et
Y¹ et Y² sont choisis de sorte que l'un, mais pas les deux, peut être H, et l'un ou les deux peuvent être indépendamment nitro ; hydrocarbyle en C₁ à C₁₄, y compris hydrocarbyle cyclique et hydrocarbyle insaturé, substitué par 1 ou 2 substituants choisis dans le groupe constitué par pyrrolidino, pipéridino, acétylaminoalkyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ substitué par des substituants pyrrolidino ou pipéridino, étant entendu que lorsque n est 1, X est H, méthyle ou éthyle, et l'un parmi Y¹ et Y² est H, l'autre parmi Y¹ et Y² n'est pas NH₂ ; ou un sel pharmacologiquement acceptable de ce composé, destinée à l'utilisation dans une méthode de traitement du corps humain ou animal pour radiosensibiliser des cellules tumorales hypoxiques.

33. Composition selon la revendication 32, dans laquelle X est os ou un groupe alcoxy en C₁ à C₄.

34. Composition selon la revendication 32, dans laquelle X est NH₂, ou NHR ou NRR.

35. Composition selon la revendication 32, dans laquelle X est NH₂.

36. Composition selon la revendication 35, dans laquelle Y¹ est H, Y² est un groupe nitro, et n est 1.

37. Composition selon la revendication 32, dans laquelle X est H.

38. Composition selon la revendication 32, dans laquelle X est un groupe hydrocarbyle en C₁ à C₄.

39. Composé ayant la formule structurale : 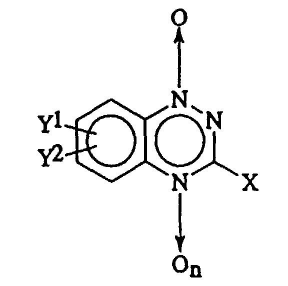 dans laquelle
X est NHR ou NRR, où chaque R est indépendamment choisi parmi un alkyle de 1 à 4 atomes de carbone substitué ou un acyle de 1 à 4 atomes de carbone substitué, ou bien, lorsque les deux groupes R sont des alkyles liés ensemble pour former un cycle pyrrolidino ou pipéridino ou liés par un oxygène pour former un cycle morpholino, et les groupes R sont substitués par des substituants alkyl (C₁ à C₄)-amino secondaire, dialkyl (C₁ à C₄)-amino tertiaire, pyrrolidino ou pipéridino ;
n est 1 ; et
Y¹ et Y² sont indépendamment H ; nitro ; halogène ; hydrocarbyle en C₁ à C₁₄, y compris hydrocarbyle cyclique et hydrocarbyle insaturé, non substitué ou substitué par 1 ou 2 substituants choisis dans le groupe constitué par halogène, hydroxy, époxy, alcoxy en C₁ à C₄, alkylthio en C₁ à C₄, amino primaire (NH₂), alkyl inférieur (C₁ à C₄)-amino secondaire, dialkyl (C₁ à C₄)-amino tertiaire, morpholino, pyrrolidino, pipéridino, acyloxy en C₁ à C₄, acylamido en C₁ à C₄ et les analogues thio de ceux-ci, acétylaminoalkyle en C₁ à C₄, carboxy, alcoxycarbonyle en C₁ à C₄, carbamyle, alkylcarbamyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ qui est non substitué ou substitué par des substituants OH, NH₂, un groupe alkyl (C₁ à C₄)-amino secondaire, dialkyl (C₁ à C₄)-amino tertiaire, morpholino, pyrrolidino, pipéridino, alcoxy en C₁ à C₄ ou halogène ;
ou un sel pharmacologiquement acceptable de ce composé.

40. Composé selon la revendication 39, dans lequel Y¹ et Y² sont tous les deux H.

41. Composé selon la revendication 39, dans lequel X est -NH-CH₂-(CH₂)ₘ-CH₂-NR₁R₂ où m est un entier dans la gamme de 0 à 4 inclusivement, et R₁ et R₂ sont indépendamment choisis parmi l'hydrogène ou les groupes alkyles inférieurs ou forment ensemble un cycle pipéridino ou pyrrolidino.

42. Composé selon la revendication 41, dans lequel m est 1 ou 2 et Y¹ et Y² sont indépendamment choisis dans le groupe constitué par H et le groupe nitro.

43. Composé ayant la formule structurale : 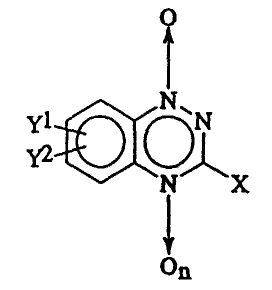 dans laquelle
X est de l'hydrogène ou un groupe hydrocarbyle en C₂ à C₄ ; ou bien X est OH, un groupe alcoxy en C₁ à C₄, NHR ou NRR, dans lesquels chaque R est indépendamment un alkyle de 1 à 4 atomes de carbone, ou un acyle de 1 à 4 atomes de carbone, ou dans lesquels les deux groupes R sont des alkyles liés ensemble pour former un cycle pyrrolidino ou pipéridino ou liés par un oxygène pour former un cycle morpholino et les groupes R peuvent être en outre substitués par des substituants OH, NH₂, alkyl (C₁ à C₄)-amino secondaire, dialkyl (C₁ à C₄)-amino tertiaire, pyrrolidino, pipéridino, alcoxy en C₁ à C₄ ou halogène ;
n est 1 ; et
Y¹ et Y² sont choisis de sorte que l'un, mais pas les deux, peut être de l'hydrogène, et l'un ou les deux peuvent être indépendamment nitro ; hydrocarbyle en C₁ à C₁₄, y compris hydrocarbyle cyclique et hydrocarbyle insaturé, ledit hydrocarbyle étant substitué par 1 ou 2 substituants choisis dans le groupe constitué par pyrrolidino, pipéridino, acétylaminoalkyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ substitué par un ou plusieurs substituants pyrrolidino ou pipéridino, étant entendu que lorsque X est H ou éthyle, et l'un parmi Y¹ et Y² est H, l'autre parmi Y¹ et Y² n'est pas NH₂ ;
ou un sel pharmacologiquement acceptable de ce composé.

44. Composé selon la revendication 43, dans lequel X est OH ou un groupe alcoxy.

45. Composé selon la revendication 43, dans lequel X est NRR.

46. Composé selon la revendication 43, dans lequel X est H.

47. Composé selon la revendication 43, dans lequel X est un groupe hydrocarbyle en C₂ à C₄.

48. Composé ayant la formule structurale : 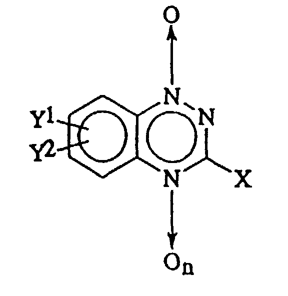 dans laquelle
X est NH₂ ; ou un groupe hydrocarbyle en C₂ à C₄ substitué par des substituants OH, NH₂, alcoxy en C₁ à C₄ ou halogène;
n est 1 ; et
Y¹ et Y² sont choisis de sorte que l'un, mais pas les deux, peut être de l'hydrogène, et l'un ou les deux peuvent être indépendamment nitro, hydrocarbyle en C₇ à C₁₄ saturé ou insaturé, ou hydrocarbyle en C₂ à C₆ insaturé, lesdits groupes hydrocarbyles étant substitués par 1 ou 2 substituants choisis dans le groupe constitué par alkyl inférieur (C₁ à C₄)-amino secondaire, dialkyl (C₁ à C₄)-amino tertiaire, pyrrolidino, pipéridino, acétylaminoalkyle en C₁ à C₄, carboxy, alcoxycarbonyle en C₁ à C₄, carbamyle, alkylcarbamyle en C₁ à C₄, alkylsulfonyle en C₁ à C₄ ou alkylphosphonyle en C₁ à C₄, le groupe hydrocarbyle pouvant être facultativement interrompu par une liaison éther (-O-) unique ; ou morpholino, pyrrolidino, pipéridino, NH₂, NHR', NR'R', O(CO)R', NH(CO)R', O(SO)R' ou O(POR')R' où R' est un groupe hydrocarbyle en C₁ à C₄ substitué par un ou plusieurs substituants alkyl (C₁ à C₄)-amino secondaire, dialkyl (C₁ à C₄)-amino tertiaire, pyrrolidino ou pipéridino ;
ou un sel pharmacologiquement acceptable de ce composé.

49. Composé selon la revendication 48, dans lequel Y¹ est H et Y² est un groupe hydrocarbyle en C₇ à C₁₄ saturé ou insaturé.

50. Composé selon la revendication 48, dans lequel Y¹ est H et Y² est un groupe hydrocarbyle en C₂ à C₆ insaturé.

51. Composé selon la revendication 48, dans lequel Y¹ est H et Y² est un groupe nitro.
